(19) Europäisches Patentamt
European Patent Office
Office européen des brevets

(11) EP 4 484 436 A1

(12) **EUROPÄISCHE PATENTANMELDUNG**

(43) Veröffentlichungstag:
**01.01.2025 Patentblatt 2025/01**

(21) Anmeldenummer: **23182872.4**

(22) Anmeldetag: **30.06.2023**

(51) Internationale Patentklassifikation (IPC):
***C07F 9/53*** (2006.01) ***A61K 6/62*** (2020.01)
***A61K 6/887*** (2020.01) ***B33Y 70/00*** (2020.01)
***B33Y 80/00*** (2015.01) ***A61K 6/16*** (2020.01)

(52) Gemeinsame Patentklassifikation (CPC):
(C-Sets verfügbar)
**A61K 6/62; A61K 6/16; A61K 6/887; B33Y 70/00; B33Y 80/00; C07F 9/5337** (Forts.)

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**KH MA MD TN**

(71) Anmelder:
- **Ivoclar Vivadent AG**
**9494 Schaan (LI)**
- **Technische Universität Wien**
**1040 Wien (AT)**

(72) Erfinder: **Moszner, Norbert, Prof. Dr.**
**9495 Triesen (LI)**

(74) Vertreter: **Uexküll & Stolberg**
**Partnerschaft von**
**Patent- und Rechtsanwälten mbB**
**Beselerstraße 4**
**22607 Hamburg (DE)**

(54) **RADIKALISCHE POLYMERISIERBARE WERKSTOFFE ZUR HERSTELLUNG DENTALER FORMKÖRPER MIT GERINGER VERFÄRBUNG**

(57) Initiatorsystem für die radikalische Polymerisation, das mindestens ein Monoacylphosphinoxid der Formel (I) in der

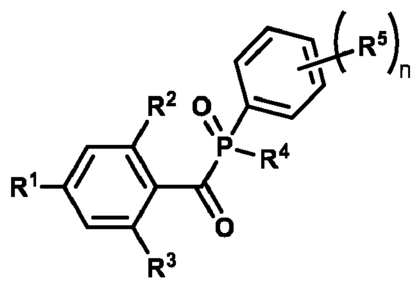

Formel (I)

$R^1$, $R^2$, $R^3$
unabhängig voneinander jeweils H, oder ein linearer oder verzweigter $C_1$-$C_{10}$-Alkylrest oder $OR^6$ sind,
$R^4$
ein linearer oder verzweigter $C_1$-$C_{10}$-Alkyl-, $C_1$-$C_{10}$-Alkoxy-, Cyclohexyl-rest oder ein Phenyl-, Methylphenyl- oder Mesityl-Rest ist,
$R^5$
ein linearer oder verzweigter $C_1$-$C_{10}$-Alkyl-Rest ist,
$R^6$
ein linearer oder verzweigter $C_1$-$C_6$-Alkyl-Rest ist, und
n
0, 1, 2 oder 3 ist,
und mindestens einen phenolischen Inhibitor enthält, wobei dass das Molverhältnis von Inhibitor zu Photoinitiator in einem Bereich von 0,002 bis 0,9 liegt. Das Initiatorsystem eignet sich besonders zur Photopolymerisation radikalisch polymerisierbarer Zusammensetzungen, die urethangruppenhaltige Monomere enthalten, und zur Herstellung von dentalen Formkörpern mit geringer Verfärbung.

EP 4 484 436 A1

(52) Gemeinsame Patentklassifikation (CPC): (Forts.)

C-Sets
**A61K 6/887, C08L 33/08;**
**A61K 6/887, C08L 33/10**

## Beschreibung

Die vorliegende Erfindung betrifft radikalisch polymerisierbare Zusammensetzungen, die sich besonders als Dentalwerkstoffe zur Herstellung von dentalen Formkörpern, wie künstlichen Zähnen, Zahnprothesen, Inlays, Onlays, Splints (Aufbissschienen), Kronen, Brücken, Verblendmaterialien und orthodontische Vorrichtungen eignen, wie z.B. Kunststoffkorrekturschienen, sog. Aligner und Positionierer.

Klassische dentale Polymerisationssysteme bestehen meistens aus einer Mischung von Monomeren, Initiatorkomponenten, Stabilisatoren und Pigmenten (J. Viohl, K. Dermann, D. Quast, S. Venz, Die Chemie zahnärztlicher Füllungskunststoffe, Carl Hanser Verlag, München-Wien1986, 21-27). Dabei werden als Monomere für den Aufbau von Polymernetzwerken meistens Mischungen von Dimethacrylaten eingesetzt (vgl. A. Peutzfeldt, Resin composites in dentistry: The monomer systems, Eur. J. Oral. Sci. 105 (1997) 97-116; N. Moszner, T. Hirt, New Polymer-Chemical Developments in Clinical Dental Polymer Materials: Enamel-Dentin Adhesives and Restorative Composites, J. Polym. Sci. Part A: Polym. Chem. 50 (2012) 4369-4402). Beispiele hierfür sind die hochviskosen Dimethacrylate 2,2-Bis[4-(2-hydroxy-3-methacryloyloxypropyl)phenyl]propan (Bis-GMA) und 1,6-Bis-[2-methacryloyloxyethoxycarbonylamino]-2,4,4-tri-methylhexan (UDMA) sowie die als Verdünnermonomere genutzten niedrigviskosen Dimethacrylate Bismethacryloyloxymethyltricyclo[5.2.1.]decan (TCDMA), Decandiol-1,10-dimethacrylat ($D_3MA$) und Triethylenglycoldimethacrylat (TEGDMA). Demgegenüber wird für Zahnprothesen hauptsächlich das monofunktionelle Monomer Methylmethacrylat (MMA) eingesetzt, das zwar niedrigviskos aber sehr flüchtig ist.

Je nach Anwendungsgebiet können weitere Additive zugesetzt werden. Zur Auslösung der radikalischen Polymerisation durch Licht werden Photoinitiatoren für den UVA-Bereich oder sichtbaren Bereich eingesetzt, die bei Bestrahlung mit UVA-Licht, meist mit einer Wellenlänge von 365 nm (Hg-Dampflampe) bis 395 nm (LED), oder Blaulicht (400-480 nm) Radikale bilden.

Prothesenmaterialien enthalten als eine wesentliche Komponente pulverförmiges Polymethylmethacrylat (PMMA), das mit MMA eine Paste bildet. Die Aushärtung erfolgt thermisch oder mit Redoxinitiatoren.

Ein wesentliches Problem bei der radikalischen Polymerisation von Methacrylaten ist der Polymerisationsschrumpf ($\Delta V_P$), d.h. die bei der Polymerisation auftretende Volumenkontraktion der eingesetzten Methacrylatmonomere, was z.B. zu einer sehr nachteiligen Randspaltbildung bei Füllungskompositen führen kann oder bei Prothesenmaterialen die Dimensionsstabilität nachteilig beeinflusst. Der Polymerisationsschrumpf $\Delta V_P$ beträgt z.B. bei reinem MMA 21,0 Vol.-%.

Ein weiterer Nachteil von PMMA- oder Dimethacrylatpolymerisaten ist die große Sprödigkeit der Materialien. Die geringe Bruchzähigkeit ist eine inhärente Eigenschaft des amorphen PMMA-Glases und wird bei durch Dimethacrylatmischungen gebildeten Polymernetzwerken vor allem durch deren unregelmäßige Netzwerkstruktur verursacht. Außerdem zeigen Methacrylate einen unvollständigen Doppelbindungsumsatz, was im Fall von Monomethacrylaten einen merklichen Restmonomergehalt bedingen und so die Biokompatibilität der Polymerisate nachteilig beeinflussen kann.

Zur Herstellung dentaler Formkörper werden zunehmend additive Fertigungsverfahren (AM: additive manufacturing) eingesetzt. Diese werden auch als generative Fertigungsverfahren oder 3D-Druck bezeichnet und unter dem Begriff "Rapid Prototyping" (RP) zusammengefasst. Hierbei werden auf der Basis computergenerierter Konstruktionsdaten (CAD-Daten) dreidimensionale Modelle oder Bauteile schichtweise kontinuierlich hergestellt. Übliche Verfahren sind die Stereolithographie (SL), selektives Lasersintern (SLS), 3-D-Printing, Fused Deposition Modelling (FDM), Ink-Jet-Printing (IJP), 3D-Plotting, Multi-Jet Modelling (MJM), Solid Freeform Fabrication (SFF), Laminated Object Manufacturing (LOM), Laser Powder Forming (LPF). Mit diesen Verfahren lassen sich Modelle, Bau- oder Formteile auch in Kleinserie kostengünstig herstellen (A. Gebhardt, Generative Fertigungsverfahren, 3. Aufl., Carl Hanser Verlag, München 2007, 77ff.).

Bei der Stereolithographie wird ein Formteil auf der Basis von CAD-Daten schichtweise aus einem flüssigen und härtbaren Monomerharz aufgebaut, wobei das Härten der Schichten durch gesteuerte Belichtung und damit durch Photopolymerisation erfolgt (vgl. I. Gibson, D.W. Rosen, B. Stucker et al., "Additive Manufacturing Technologies", Band 238, Springer Verlag (2010)). Hierzu wird häufig ein UV-Laser als Lichtquelle verwendet. Beim DLP-Verfahren (**D**igital **L**ight **P**rocessing) verwendet man ein projizierbares Bild zur schichtenweisen Aushärtung der Werkstoffe.

Baumaterialien zur generativen Herstellung von dentalen Formkörpern müssen im Hinblick auf die Besonderheiten dieses Verfahrens unterschiedliche Anforderungen erfüllen. Sie sollen eine niedrige Viskosität und eine hohe Transparenz aufweisen und nach der Härtung gute mechanische Eigenschaften haben. Die hergestellten Formkörper sollen außerdem eine gute Biegefestigkeit, ein hohes E-Modul und insbesondere eine gute Bruchzähigkeit aufweisen.

Zur Verbesserung der Bruchzähigkeit können Polymerisationsharzen sog. Schlagzähmodifikatoren zugesetzt werden. Hierbei handelt es sich in der Regel um Polymerpartikel mit einer Kern-Schale-Struktur.

Die EP 3 020 361 A1 offenbart radikalisch polymerisierbare Zusammensetzungen, die sich zur Herstellung von Dentalprodukten durch generative Fertigungsverfahren eigenen sollen und die mit radikalisch polymerisierbaren Gruppen substituierte Polysiloxane, Disiloxane und ggf. weiter Monomere enthalten, beispielsweise Urethan(meth)acrylatester. Sie sollen sich durch eine geringe Schrumpfung und hohe Dimensionsgenauigkeit auszeichnen.

Die US 2018/0000570 A1 betrifft Baumaterialien auf der Basis von mono- und multifunktionellen (Meth)acrylaten zur generativen Herstellung dentaler Bauteile. Die Baumaterialien enthalten Gummipartikel auf Silikon-Acryl-Basis mit Kern-Schale-Struktur (Produkt S2006, Mitsubishi Rayon Co.) als Schlagzähmodifikatoren und Oligomere, die durch Umsetzung von Trimethyl-1,6-diisocyanat, Bisphenol-A-propoxylat und 2-Hydroxyethylmethacrylat (HEMA) hergestellt werden. Die gehärteten Bauteile sollen gute mechanische und physikalische Eigenschaften sowie eine gute Bioverträglichkeit aufweisen.

Die US 10,299,896 B2 und die US 2019/0053883 A1 offenbaren durch generative Verfahren hergestellte dentale Bauteile, die mindestens zwei Schichten aus unterschiedlich zusammengesetzten Baumaterialien aufweisen. Dabei wird eine Schicht durch ein Material gebildet, das Oligomere, die durch Reaktion von Zwischenprodukten mit endständigen Isocyanatgruppen mit hydroxylbasierten Methacrylaten erhalten werden, eine polymerisierbare Acrylverbindung und einen Schlagzähmodifikator enthält. Mindestens eine weitere Schicht wird durch ein Material gebildet, das ein Urethanmonomer, ein Glycoldimethacrylat und Füllstoff enthält. Die Kombination von Materialen mit unterschiedlichen mechanischen und physikalischen Eigenschaften soll zur Anpassung der Bauteile an unterschiedliche Anforderungen vorteilhaft sein. Als Schlagzähmodifikatoren werden handelsübliche Polymere mit Kern-Schale-Struktur verwendet, wie z.B. das Produkt M570 der Firma Kaneka.

Die EP 3 564 282 A1 offenbart härtbare Zusammensetzungen für Hochtemperatur-Photopolymerisationsverfahren, die ein oligomeres Urethandimethacrylat als Glasübergangstemperaturmodifikator, ein (Poly-)Carbonat-(Poly-)-Urethan-Dimethacrylat als Zähigkeitsmodifikator und ggf. Core-Shell-Partikel enthalten. Sie sollen gute thermomechanische Eigenschaften und eine gute Bioverträglichkeit aufweisen und sich zur Herstellung kieferorthopädischer Vorrichtungen eignen.

Ein wesentlicher Nachteil für dentale Anwendungen ist, dass Kern-Schale-Polymere (CSP) die Transparenz der Materialien deutlich vermindern, was sich nachteilig auf den stereolithographischen Bauprozess auswirkt. Eine Verringerung der Transparenz reduziert die Durchhärtungstiefe und kann so zu einer Verschlechterung der mechanischen Eigenschaften und einer Erhöhung des Restmonomergehalts führen.

Aus der EP 4 085 893 A1 sind radikalisch polymerisierbare Materialien zur Herstellung dentaler Formkörper durch generative Verfahren bekannt, die zur Verbesserung der Bruchzähigkeit und Brucharbeit ein ABA- oder AB-Blockcopolymer enthalten. Die Materialien enthalten vorzugsweise außerdem 30 bis 70 Gew.-% mindestens eines monofunktionellen, radikalisch polymerisierbaren Monomers und mindestens ein radikalisch polymerisierbares Urethandi(meth)acrylattelechel mit einer zahlenmittleren Molmasse von 750 bis 2000 g/mol. Die Werkstoffe können zur Verbesserung der Bruch- und Schlagzähigkeit Kern-Schale-Polymere enthalten. Sie zeichnen sich durch einen relativ hohen Gehalt an monofunktionellen Monomeren aus.

Urethangruppenhaltige Monomere zeichnen sich durch eine sehr gute Photoreaktivität aus und ergeben Photopolymerisate mit sehr guten mechanischen Eigenschaften auch nach Wasserlagerung. Nachteilig ist, dass sie zu starken Verfärbung der daraus hergestellten Formkörper führen können. Die Verfärbungen bilden sich sowohl während der Härtung der Harze als auch bei der Lagerung der Formkörper.

Auch die zur Härtung verwendeten Photoinitiatoren können Verfärbungen verursachen. Insbesondere das für dentale Anwendungen häufig eingesetzte Campherchinon und Campherchinon-Amin-Photoinitiatorsysteme führen regelmäßig zu deutlich sichtbaren Geldverfärbungen.

Die US 2008/0009557 A1 schlägt die Zugabe von Iodoniumsalzen, wie z.B. [4-(2-Hydroxy-1-tetradecyloxy) phenyl]phenyliodoniumhexafluoroantimonat, zur Verhinderung Campherchinon-bedingter Verfärbungen vor. Die Iodoniumsalze sollen Verfärbungen durch Oxidation der bei der Polymerisation entstehenden, gelben Nebenprodukte verringern.

T. Sumiyoshi, W. Schnabel, W.; Henne, A., "Photolysis of acylphosphine oxides II: The influence of methyl substitution in benzoyldiphenylphosphine oxides", J. Photochem. 32 (1986) 119, untersuchen den Einfluss der Substitution von Wasserstoffatomen der Benzoylgruppe von Benzoyldiphenylphosphinoxiden durch Methylgruppen auf die lichtinduzierte $\alpha$-Spaltung der Benzoyldiphenylphosphinoxide. Es wurde gefunden, dass die Substitution des para-Wasserstoffs die Spaltung am deutlichsten beschleunigt, während das dreifach substituierte 2,4,6-Trimethylbenzoyldiphenylphosphinoxid in etwa die gleiche Spaltungsrate wie das unsubstituierte Benzoyldiphenylphosphinoxid zeigte. Die Substitution in ortho-Position verlangsamte die Spaltung.

C. Dietlin, T. T. Trinh, S. Schweizer, B. Graff, F. Morlet-Savary, P.-A. Noirot, J. Lalevée, "Rational design of acyldiphenylphosphine oxides as photoinitiators of radical polymerization", Macromolec. 52 (2019) 7886), vergleichen die Eigenschaften von Monoacylphosphinoxiden mit unterschiedlichen Substituenten an der Benzoylgruppe mit den anhand von theoretischen Berechnungen vorhergesagten Eigenschaften. Es wurde eine gute Übereistimmung von gemessenen und berechneten Werten gefunden. Die Photoinitiatorreaktivität und der Doppelbindungsumsatz korrelieren stark mit der Löslichkeit der Monoacylphosphinoxide.

H. Duan, K. Leng, X. Xu, Q. Li, D. Liu, Y. Han, J. Gao, Q. Yu, Z. Wang, "Monoacylphosphine oxides with substituents in the phosphonyl moiety as Norrish I photoinitiators: Synthesis, photoinitiation properties and mechanism", J. Photochem. Photobiol. A: Chem. 421 (2021) 113517, untersuchen die Wirkung von Substituenten an der Phosphonylgruppe von Monoacylphosphinoxiden auf die Stabilität und Initiatoreigenschaften. Es wurde gefunden, dass sowohl die Stabilität als auch die Lichtabsorption durch die Einführung von Methylgruppen verbessert werden können.

Im Vergleich zum Campherchinon weisen Monoacylphosphinoxide und Bisacylphosphinoxide zwar nur eine geringe Eigenfarbe auf, führen aber insbesondere in Kombination mit Urethan(meth)acrylaten ebenfalls häufig zu Gelbverfärbungen. Diese entstehen durch Spaltprodukte der Photoinitiatoren und deren Folgeprodukte, wie z.B. Kombinationsprodukte. Diese farbigen Verunreinigungen werden bei der Lagerung der Formkörper unter feuchten Bedingungen an Tageslicht mit der Zeit zwar abgebaut, gewöhnlich erfordert dieser Vorgang aber Zeiträume von mehreren Wochen bis zu einigen Monaten. Für die praktische Anwendung als Dentalmaterial sind aber Werkstoffe erwünscht, die sich nach wenigen Tagen so weit entfärben, dass mit dem bloßen Auge keine Verfärbungen mehr sichtbar sind.

Der Erfindung liegt die Aufgabe zugrunde, polymerisierbare Materialien zur Herstellung dentaler Formkörper bereitzustellen, farbstabil sind und sich bei der Polymerisation nicht verfärben oder sich danach zumindest rasch entfärben. Die Materialien sollen sich zur Verarbeitung mit generativen Verfahren eignen und nach der Härtung gute mechanische Eigenschaften aufweisen, insbesondere eine hohe Bruchzähigkeit, eine gute Biegefestigkeit und ein relativ hohes Biegemodul, gut bioverträglich und schnell härtbar sein.

Die Aufgabe wird erfindungsgemäß durch ein Initiatorsystem für die radikalische Polymerisation gelöst, das mindestens ein Monoacylphosphinoxid der Formel (I) in der

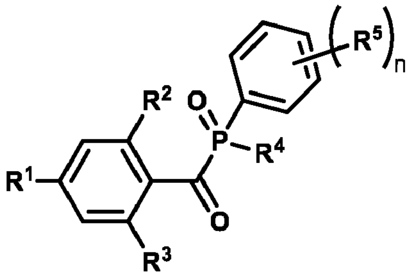

Formel (I)

| | |
|---|---|
| $R^1$, $R^2$, $R^3$ | unabhängig voneinander jeweils H, oder ein linearer oder verzweigter $C_1$-$C_{10}$-Alkylrest oder $OR^6$ sind, |
| $R^4$ | ein linearer oder verzweigter $C_1$-$C_{10}$-Alkyl-, $C_1$-$C_{10}$-Alkoxy-, Cyclohexyl-rest oder ein Phenyl-, Methylphenyl- oder Mesityl-Rest ist, |
| $R^5$ | ein linearer oder verzweigter $C_1$-$C_{10}$-Alkyl-Rest ist, |
| $R^6$ | ein linearer oder verzweigter $C_1$-$C_6$-Alkyl-Rest ist, und |
| n | 0, 1, 2 oder 3 ist, |

und mindestens einen phenolischen Inhibitor enthält. Das Initiatorsystem ist dadurch gekennzeichnet, dass das Molverhältnis von Inhibitor zu Photoinitiator in einem Bereich von 0,002 bis 0,9, vorzugsweise 0,02 bis 0,8, besonders bevorzugt 0,05 bis 0,2 liegt.

Bevorzugt sind Photoinitiatoren der Formel (I) bei denen die Variablen die folgenden Bedeutungen haben:

| | |
|---|---|
| $R^1$, $R^2$, $R^3$ | unabhängig voneinander jeweils ein linearer oder verzweigter $C_1$-$C_3$-Alkylrest oder $OR^6$, bevorzugt Methyl oder Methoxy, |
| $R^4$ | ein linearer oder verzweigter $C_1$-$C_5$-Alkyl-, $C_1$-$C_5$-Alkoxy-, Cyclohexyl- oder Phenyl-Rest, bevorzugt Ethoxy, Cyclohexyl oder Phenyl, |
| $R^5$ | ein linearer oder verzweigter $C_1$-$C_3$-Alkyl-Rest, bevorzugt Methyl, |
| $R^6$ | ein linearer oder verzweigter $C_1$-$C_3$-Alkyl-Rest, bevorzugt Ethyl, und |
| n | 0, 1 oder 3, bevorzugt 0. |

Gemäß einer weiteren bevorzugten Ausführungsform haben die Variablen der Formel (I) die folgenden Bedeutungen:

| | |
|---|---|
| $R^1$, $R^2$, $R^3$ | unabhängig voneinander jeweils H, ein linearer oder verzweigter $C_1$-$C_3$-Alkylrest oder $OR^6$, bevorzugt H, Methyl oder Methoxy, wobei besonders bevorzugt zwei der Reste $R^1$, $R^2$ und $R^3$ Methoxy sind und ein Rest H oder Methyl ist, |
| $R^4$ | ein linearer oder verzweigter $C_1$-$C_5$-Alkyl-, $C_1$-$C_5$-Alkoxy-, Cyclohexyl- oder Phenyl-Rest, bevorzugt tert,-Butyl, Ethoxy, Cyclohexyl oder Phenyl, |
| $R^5$ | ein linearer oder verzweigter $C_1$-$C_3$-Alkyl-Rest, bevorzugt Methyl, |
| $R^6$ | ein linearer oder verzweigter $C_1$-$C_3$-Alkyl-Rest, bevorzugt Methyl, und |

n 0, 1, 2 oder 3, ganz besonders bevorzugt 3,

wobei dann, wenn $R^4$ kein Phenylrest ist, n vorzugsweise 0 ist, und wenn $R^4$ ein Phenylrest ist, n vorzugsweise 1, 2 oder 3 ist.

Weiter bevorzugt sind Verbindungen der Formel (I), in der die Variablen die folgenden Bedeutungen haben:

$R^1$ H, ein linearer oder verzweigter $C_1$-$C_3$-Alkylrest oder $OR^6$, bevorzugt H, Methyl oder Methoxy,

$R^2$, $R^3$ unabhängig voneinander jeweils H, ein linearer oder verzweigter $C_1$-$C_3$-Alkylrest oder $OR^6$, bevorzugt H, Methyl oder Methoxy, besonders bevorzugt Methyl oder Methoxy und am meisten bevorzugt Methoxy,

$R^4$ ein linearer oder verzweigter $C_1$-$C_5$-Alkyl-, $C_1$-$C_5$-Alkoxy-, Cyclohexyl- oder Phenyl-Rest, bevorzugt Ethoxy, Cyclohexyl oder Phenyl,

$R^5$ ein linearer oder verzweigter $C_1$-$C_3$-Alkyl-Rest, bevorzugt Methyl,

$R^6$ ein linearer oder verzweigter $C_1$-$C_3$-Alkyl-Rest, bevorzugt Ethyl, und

n 0, 1 oder 3, bevorzugt 0.

Besonders bevorzugt ist mindestens einer der Reste $R^1$, $R^2$ und $R^3$ $OR^6$, und gemäß einer ganz besonders bevorzugten Ausführungsform sind 2 oder 3 der Reste $R^1$ bis $R^3$ $OR^6$. Vorzugsweise ist zumindest $R^1$ $OR^6$. Derartige Photoinitiatoren zeichnen sich durch eine besonders gute Farbstabilität aus.

Es wurde überraschend gefunden, dass Photoinitiatoren gemäß Formel (I) in Kombination mit einer definierten Menge ausgewählter Inhibitoren die Herstellung von radikalisch polymerisierbaren Zusammensetzungen ermöglichen, die lagerstabil sind und nach der Polymerisation keine Verfärbungen aufweisen, ohne dass die Inhibitoren die Polymerisation beeinträchtigen. Besonders vorteilhaft ist, dass auch Zusammensetzungen mit radikalisch polymerisierbaren, urethangruppenhaltigen Monomeren ohne Verfärbungen gehärtet werden können.

Radikalisch polymerisierbare Zusammensetzungen, die neben dem erfindungsgemä-ßen Initiatorsystem mindestens ein radikalisch polymerisierbares, urethangruppenhaltiges Monomer enthalten, sind ebenfalls Gegenstand der Erfindung.

Besonders bevorzugt sind Zusammensetzung, die

(a) 0,1 bis 8 Gew.-% mindestens eines Monoacylphosphinoxids der Formel (I),

(b) 1 bis 99 Gew.-% mindestens eines urethangruppenhaltigen Monomers, vorzugsweise Urethandi(meth)acrylats,

(c) 0 bis 70 Gew.-% eines oder mehrerer aliphatischer, cycloaliphatischer, aromatischer, bicyclischer oder tricyclischer Mono(meth)acrylate,

(d) 0 bis 50 Gew.-% eines oder mehrerer Di(meth)acrylats ohne Urethangruppen,

(e) 0 bis 12 Gew.-% eines oder mehrerer Blockcopolymere und/oder Kern-Schale-Polymeren,

(f) 200 bis 1200 ppm, vorzugsweise 300 bis 1200 ppm, mindestens eines phenolischen Inhibitors,

(g) 0 bis 50 Gew.-% an anorganischen oder organischen Füllstoffen,

(h) 0 bis 30 Gew.-% an weiteren Additiven enthalten.

Alle Gewichtsprozentangaben hierin beziehen sich jeweils auf die Gesamtmasse der Zusammensetzung, wenn nicht anders angegeben.

Die Menge an Monoacylphosphinoxiden der Formel (I) wird vorzugsweise so gewählt, dass sie im Bereich von 0,1 bis 6,0 Mol.-% bezogen auf den Doppelbindungsgehalt der radikalisch polymerisierbaren Bestandteile liegt.

Ganz besonders bevorzugt sind Zusammensetzungen, die

(a) 0,30 bis 6,5 Gew.-%, vorzugsweise 0,5 bis 4,0 Gew.-% und ganz besonders bevorzugt 0,9 bis 2,6 Gew.-% mindestens eines Monoacylphosphinoxids der Formel (I),

(b) 1 bis 80 Gew.-%, vorzugsweise 10 bis 75 Gew.-%, mindestens eines urethangruppenhaltigen Monomers, vorzugsweise Urethandi(meth)acrylats,

(c) 0 bis 66 Gew.-%, vorzugsweise 0 bis 60 Gew.-%, besonders bevorzugt 0 bis 5 Gew.-% oder 5 bis 60 Gew.-%, vorzugweise 40 bis 60 Gew.-%, eines oder mehrerer aliphatischer, cycloaliphatischer, aromatischer, bicyclischer oder tricyclischer Mono(meth)acrylate,

(d) 0 bis 40 Gew.-%, vorzugsweise 0 bis 25 Gew.-%, eines oder mehrerer Di(meth)acrylate ohne Urethangruppen,

(e) 0 bis 10 Gew.-%, vorzugsweise 0 bis 8 Gew.-%, besonders bevorzugt 0 bis 2 Gew.-% oder 4 bis 8 Gew,-% eines oder mehrerer Blockcopolymere und/oder Kern-Schale-Polymere,

(f) 300 bis 1000 ppm, vorzugsweise 300 bis 500 ppm, mindestens eines phenolischen Inhibitors,

(g) 0 bis 40 Gew.-%, vorzugsweise 0 bis 25 Gew.-%, an anorganischen und/oder organischen Füllstoffen,

(h) 0 bis 25 Gew.-%, vorzugsweise 0,01 bis 10 Gew.-%, eines oder mehrerer weiterer Additive, die aus UV-Absorbern, optischen Aufhellern, Farbmitteln, Weichmachern und Thixotropieadditiven ausgewählt sind, enthalten. Die Mengenangabe für die Additive bezieht sich auf die Gesamtmasse an Additiven.

Die Menge an Monoacylphosphinoxiden der Formel (I) wird im Fall der ganz besonders bevorzugten Ausführungsform vorzugsweise so gewählt, dass sie im Bereich von 0,3 bis 5,0 Mol.-%, vorzugsweise 0,4 bis 3,0 Mol.-% und ganz besonders bevorzugt 0,7 bis 2,0 Mol.-% bezogen auf den Doppelbindungsgehalt der radikalisch polymerisierbaren Bestandteile liegt.

Erfindungsgemäß bevorzugte Monoacylphosphinoxide der Formel (I) sind:

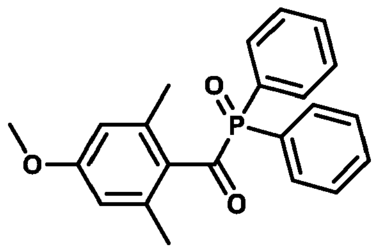
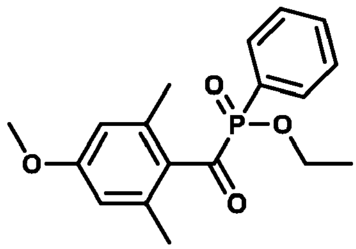
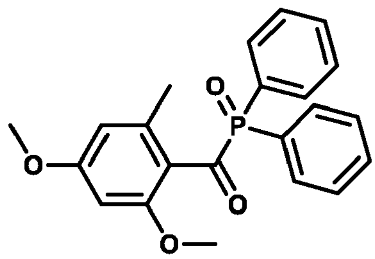

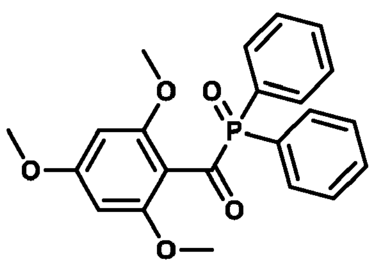
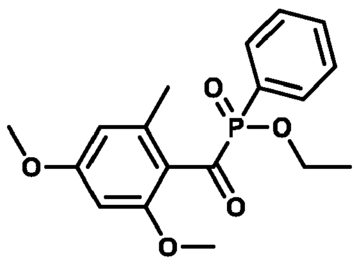
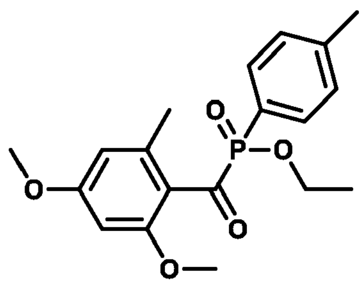

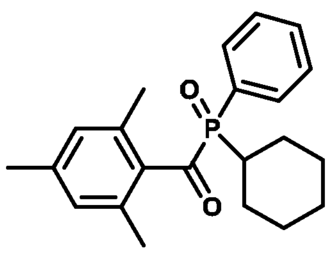
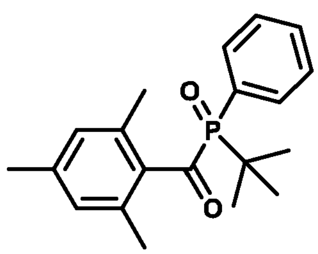
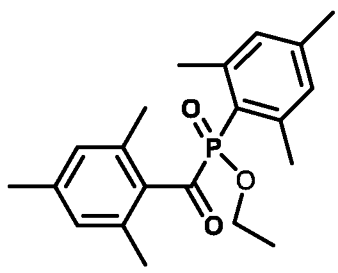

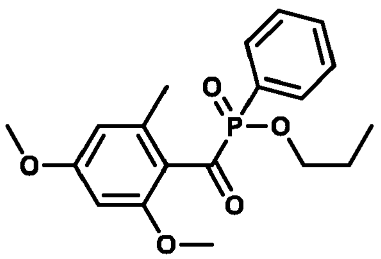

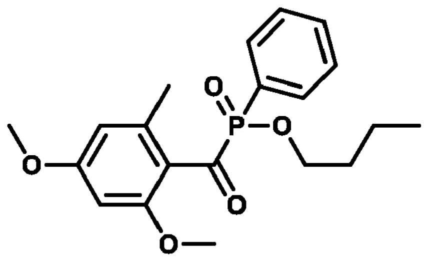

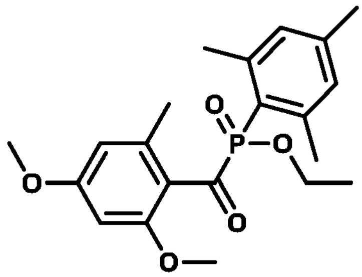

Monoacylphosphinoxide der Formel (I) lassen sich nach den bekannten Methoden der organischen Chemie herstellen, beispielsweise durch eine Eintopfsynthese bei der in einem 1. Schritt ein entsprechend substituierter Benzaldehyd in Gegenwart einer Base, z.B. Triethylamin oder Natriumcarbonat, z.B. in Tetrahydrofuran (THF) als Lösungsmittel mit einem entsprechenden Phosphinoxid bei Raumtemperatur oder erhöhter Temperatur innerhalb von Stunden oder einigen Tagen umgesetzt wird:

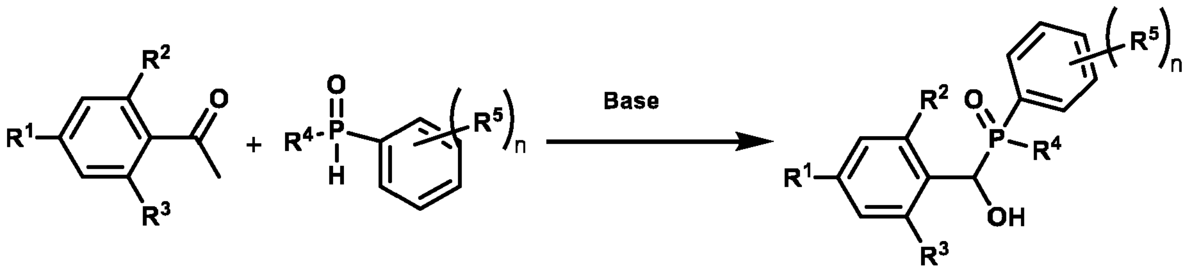


In einem 2. Schritt wird nach Entfernen des Lösungsmittels unter Lichtausschluss Mangan(IV)-oxid als Oxidationsmittel in Dichlormethan (DCM) zugegeben und dann nach Stunden oder Tagen das entsprechende Monoacylphosphinoxid der Formel (I) erhalten:

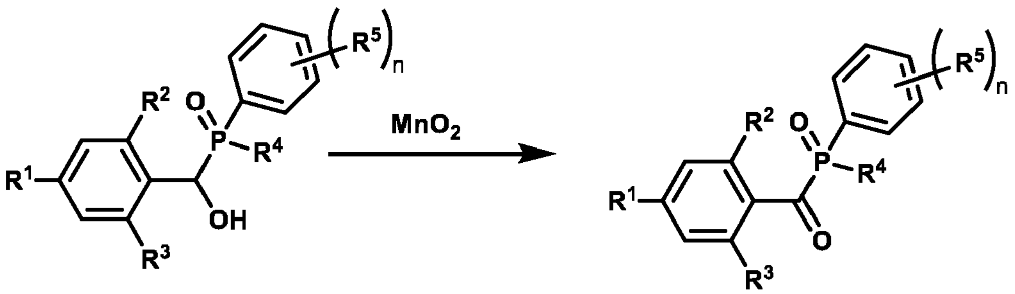


Konkretes Beispiel:

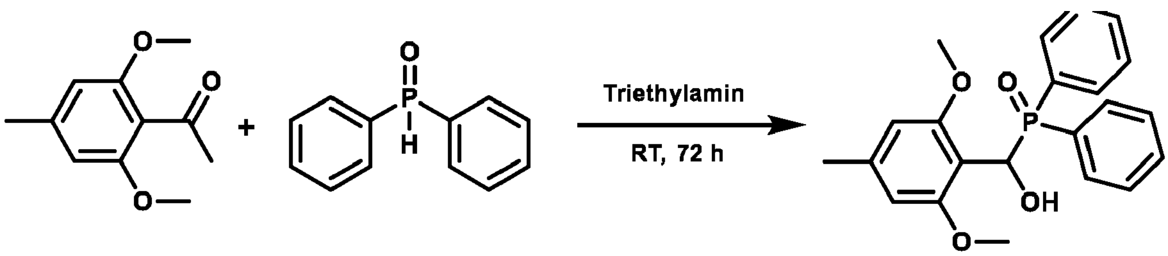

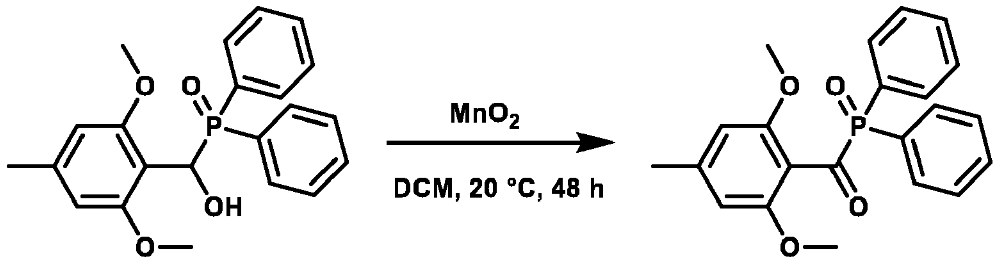


Als **urethangruppenhaltiges (Meth)acrylatmonomer (b)** wird vorzugsweise ein Monomer oder eine Mischung von Monomeren verwendet, das oder die einen Urethangruppengehalt von 0,01 bis 5 mmol/g, vorzugsweise 0,1 bis 4,5 mmol/g und besonders bevorzugt 0,5 bis 4,0 mmol/g aufweist/aufweisen. Besonders bevorzugt sind Urethan-di(meth) acrylate, ganz besonders Urethandi(meth)acrylate mit einer Molmasse von kleiner 4000 g/mol und am meisten bevorzugt Urethandi(meth)acrylate mit einer Molmasse von kleiner 4000 g/mol, die 1 bis 8, vorzugsweise 1 bis 6 und besonders bevorzugt 1 bis 4 Urethangruppen enthalten.

Erfindungsgemäß bevorzugte urethangruppenhaltige (Meth)acrylatmonomere sind erhältlich durch:

A) Reaktion von 2 Mol 2-Isocyanatoethyl(meth)acrylat ($R^8$: H oder $CH_3$) mit einem Mol an $\alpha,\omega$-Alkandiolen, cycloaliphatischen, tricyclischen oder aromatischen Diolen sowie ethoxylierten oder propoxylierten aromatischen Diolen ($R^7$: $C_1$-$C_{20}$ aliphatisches, cycloaliphatisches oder tricyclisches Alkylen oder $C_6$-$C_{14}$-Arylen bzw. alkoxyliertes $C_6$-$C_{14}$-Arylen):

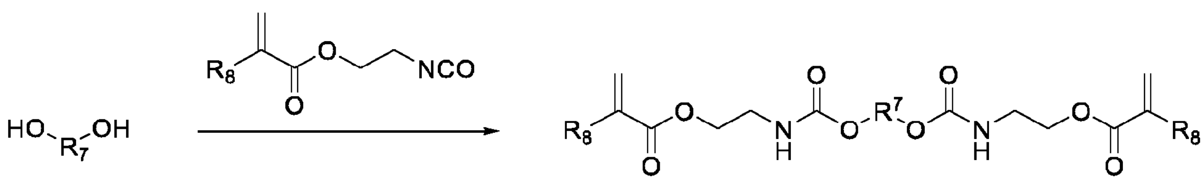

B) Reaktion von 1 Mol an einem oder mehreren Diisocyanaten ($R^9$: $C_1$-$C_{12}$ aliphatisches, cycloaliphatisches Alkylen oder $C_6$-$C_{14}$-Arylen, die Alkylsubstituenten tragen können) mit 2 Mol 2-Hydroxyalkyl(meth)acrylat, Poly(ethylenglycol)- oder Poly(propylenglycol)mono(meth)acrylat sowie Mischungen davon ($R^{10}$: H oder $CH_3$; $R^{11}$: lineares oder verzweigtes $C_1$-$C_{40}$-Alkylen, wobei die Alkylenkette durch ein oder mehrere O-Atome unterbrochen sein kann):

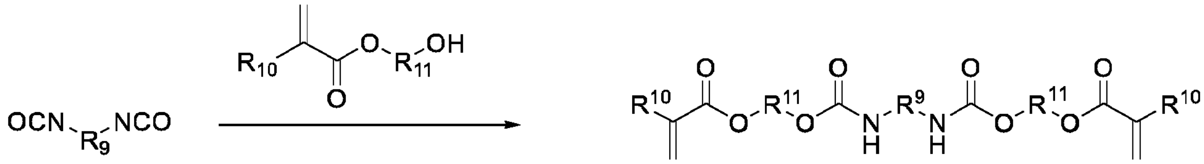

C) Reaktion von 1 Mol an ethoxyliertem oder propoxyliertem Bisphenol-A, Decandiol oder Dodecandiol oder anderen $\alpha,\omega$-Alkandiolen, cycloaliphatischen, tricyclischen oder aromatischen Diolen bzw. ethoxy- bzw. propoxylierten aromatischen Diolen ($R^7$: $C_1$-$C_{20}$ aliphatisches, cycloaliphatisches oder tricyclisches Alkylen oder $C_6$-$C_{14}$-Arylen bzw. alkoxyliertes $C_6$-$C_{14}$-Arylen),) mit 2 Mol Isophorondiisocyanat (IPDI) und anschließender Umsetzung mit 2 Mol 2-Hydroxyethyl(meth)acrylat oder Hydroxypropyl(meth)acrylat ($R^{10}$: H oder CHs; $R_7$: lineares oder verzweigtes $C_1$-$C_{40}$-Alkylen, wobei die Alkylenkette durch ein oder mehrere O-Atome unterbrochen sein kann):

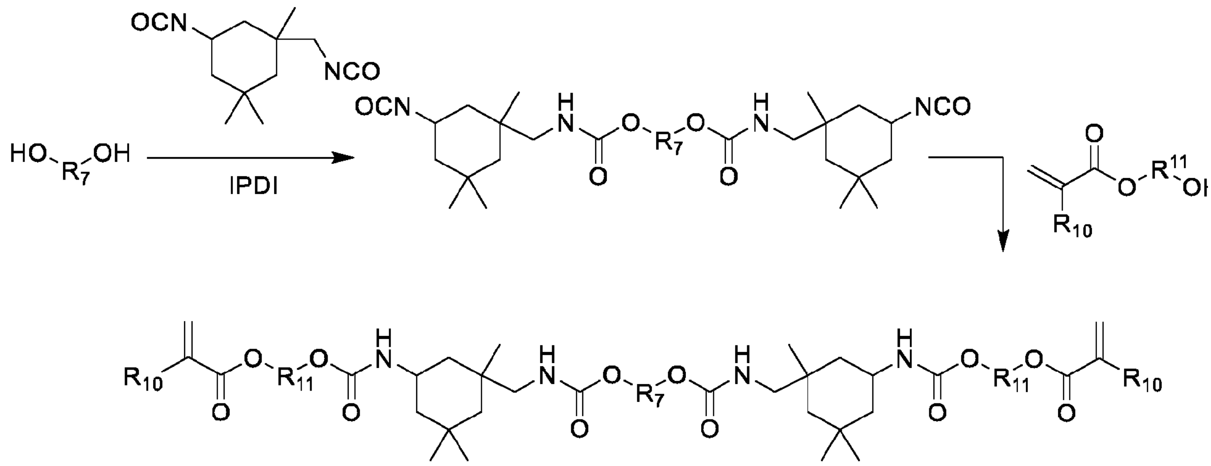


D) Reaktion von 1 Mol 2-Isocyanatoethyl(meth)acrylat mit 1 Mol 2-Hydroxyalkyl-(meth)acrylat, Poly(ethylenglycol)- oder Poly(propylenglycol)mono(meth)acrylat ($R^{11}$: lineares oder verzweigtes $C_1$-$C_{40}$-Alkylen, wobei die Alkylenkette durch ein oder mehrere O-Atome unterbrochen sein kann; $R^8$, $R^{10}$: H oder CHs):

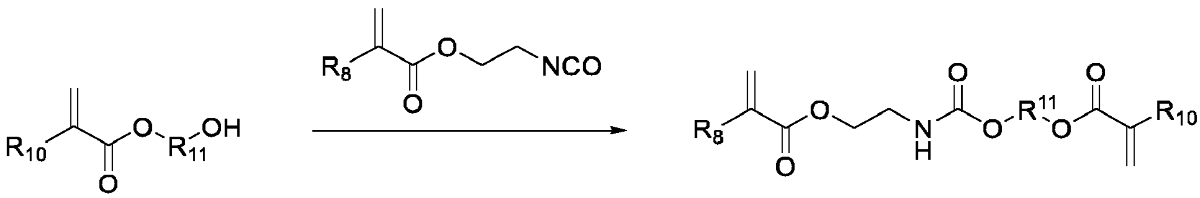

Erfindungsgemäß besonders bevorzugt sind Urethandi(meth)acrylate mit eine Molmasse kleiner 750 g/mol, die gemäß Syntheseweg A erhältlich sind durch:
Reaktion von 2 Mol 2-Isocyanatoethyl(meth)acrylat mit einem Mol an $\alpha,\omega$-Alkandiolen (Ethylenglycol, Propylenglycol, Butan-1,4-diol, Pentan-1,5-diol, Neopentylglycol, Hexan-1,6-diol, 2,2,4-Timethylhexandiol, 2,2,4-Trimethyl-1,3-pentandiol, Heptan-1,7-diol, Octan-1,8-diol), Nonandiol-1,9-diol, Decan-1,10-diol, Undecan-1,11-diol oder Dodecan-1,12-diol), $C_6$-$C_{18}$-Alkandiole, die 1 bis 4 O- oder S-Atome in der Kohlenstoffkette enthalten können, cycloaliphatische Diole (2-Methyl-1,4-cyclopentandiol, 1,3- oder 1,4-Cyclohexandiol, Cyclooctandiol, Methylcycloheptandiol, 2,5-Dimethyl-1,4-cyclohexandiol, 3,3,5-Trimethylcyclopentane-1,2-diol, 2,2,4,4-Tetramethyl-1,3-cyclobutandiol, tricyclischen (Tricyclodecandimethanol: Tricyclo[5.2.1.$0^{2,6}$]decandimethanol) oder aromatischen ethoxy- bzw. propoxylierten Diolen mit im Mittel 2, 3 oder 4 Ethoxy- oder Propoxygruppen (Bisphenol-A, Bisphenol-B, Bisphenol-E, Bisphenol-F, Bisphenol-S, Hydrochinon, Resorcin, 2,6-Dihydroxynaphthalin oder 4,4'-Dihydroxybiphenyl).

Ebenso besonders bevorzugt sind Urethandi(meth)acrylate mit eine Molmasse kleiner 750 g/mol, die gemäß Syntheseweg B erhältlich sind durch:
Reaktion von 1 Mol an Hexamethylen-1,6-diisocyanat (HMDI), 2,2,4-Trimethylhexamethylen-1,6-diisocyanat (TMDI), Isophorondiisocyanat (IPDA), Diphenylmethan-4,4'-diisocyanat 4,4'-MDI), hydriertem Diphenylmethan-4,4'-diisocyanat ($H_{12}$-MDI: 4,4'-Methylen-bis-(cyclohexylisocyanat) oder Tetramethylxylylendiisocyanat (TMXDI: 1,3-Bis(2-isocyanatopropan-2-yl)benzol) bzw. deren Mischungen mit 2 Mol 2-Hydroxyethyl(meth)acrylat, Hydroxypropyl(meth)acrylat oder Poly(ethylenglycol)-200- bzw. Poly(propylenglycol)-200-mono(meth)acrylat.

Als urethangruppenhaltige (Meth)acrylatmonomere (b) sind weiterhin Urethandi(meth)acrylate mit einer Molmasse kleiner 1000 g/mol bevorzugt, die beispielsweise durch Reaktion eines Diisocyanats mit 2 Mol eines hydroxylgruppenhaltigen (Meth)acrylats oder durch Reaktion eines Diols mit 2 Mol eines isocyanatgruppenhaltigen (Meth)acrylats erhältlich sind.

Besonders bevorzugt sind Urethandi(meth)acrylate mit einer Molmasse kleiner 1000 g/mol, die durch Umsetzung von 1 Mol an Hexamethylen-1,6-diisocyanat (HMDI), 2,2,4-Trimethylhexamethylen-1,6-diisocyanat (TMDI), Isophorondiisocyanat (IPDA), Diphenylmethan-4,4'-diisocyanat 4,4'-MDI), hydriertem Diphenylmethan-4,4'-diisocyanat ($H_{12}$-MDI: 4,4'-Methylen-bis-(cyclohexylisocyanat) oder Tetramethylxylylendiisocyanat (TMXDI: 1,3-Bis(2-isocyanatopropan-2-yl)benzol), oder einer Mischung davon, mit 2 Mol 2-Hydroxyethyl(meth)acrylat, Hydroxypropyl(meth)acrylat, Poly(ethylenglycol)-200- oder Poly(propylenglycol)-200-mono(meth)acrylat erhältlich sind.

Ebenso besonders bevorzugt sind Urethandi(meth)acrylate mit einer Molmasse kleiner 1000 g/mol, die durch Umsetzung von 2 Mol 2-Isocyanatoethyl(meth)acrylat mit einem Mol eines $\alpha,\omega$-Alkandiols, vorzugsweise Ethylenglycol, Propylenglcol, Butan-1,4-diol, Pentan-1,5-diol, Neopentylglycol, Hexan-1,6-diol, 2,2,4-Timethylhexandiol, 2,2,4-Trimethyl-1,3-pentandiol, Heptan-1,7-diol, Octan-1,8-diol, Nonandiol-1,9-diol, Decan-1,10-diol, Undecan-1,11-diol oder Dodecan-1,12-diol, eines $C_6$-$C_{18}$-Alkandiols, das 1 bis 4 O- oder S-Atome in der Kohlenstoffkette enthält, eines cycloaliphatischen Diols, vorzugsweise 2-Methyl-1,4-cyclopentandiol, 1,3- oder 1,4-Cyclohexandiol, Cyclooctandiol, Methylcycloheptandiol, 2,5-Dimethyl-1,4-cyclohexandiol, 3,3,5-Trimethyl-cyclopentane-1,2-diol oder 2,2,4,4-Tetramethyl-1,3-cyclobutandiol, eines tricyclischen Diols, vorzugsweise Tricyclodecandimethanol oder Tricyclo[5.2.1.$0^{2,6}$]decandimethanol, eines aromatischen ethoxy- oder propoxylierten Diols mit im Mittel 2, 3 oder 4 Ethoxy- oder Propoxygruppen, vorzugsweise Bisphenol-A, Bisphenol-B, Bisphenol-E, Bisphenol-F, Bisphenol-S, Hydrochinon, Resorcin, 2,6-Dihydroxynaphthalin, 4,4'-Dihydroxybiphenyl, oder einer Mischung davon, erhältlich sind.

Weitere bevorzugte Urethandi(meth)acrylate sind die in EP 4 085 893 A1 offenbarten Urethandi(meth)acrylat-Telechele. Diese sind durch Umsetzung von Diisocyanaten mit Diolen (HO-DA-OH) und anschließender Reaktion der $\alpha,\omega$-isocyanat-funktionalisierten Urethantelechele mit 2-Hydroxyethylmethacrylat (HEMA) oder Hydroxypropylmethacrylat (HPMA) erhältlich. DA steht für einen aromatischen oder aliphatischen Kohlenwasserstoffrest mit 6 bis 33 Kohlenstoffatomen, vorzugsweise einen divalenten polycyclischen Kohlenwasserstoffrest, insbesondere einen o-Diphenyl-, p-Diphenyl- oder Bisphenol A-Rest, oder eine verzweigte oder vorzugsweise lineare $C_2$-$C_{18}$-Alkylengruppe. Die Kohlenwasserstoffreste können ein oder mehrere O-Atome und/oder S-Atome enthalten, wobei O-Atome bevorzugt sind.

Bevorzugt sind Telechele mit einer Molmasse von 800 bis 4000 g/mol, besonders bevorzugt 1000 bis 3800 g/mol und ganz besonders bevorzugt 1000 bis 2000 g/mol. Weiter bevorzugt sind Telechele, die 1 bis 8, besonders bevorzugt 4 bis 6, Urethangruppen enthalten.

Bevorzugte Diole der Formel HO-DA-OH sind ethoxyliertes- oder propoxyliertes Bisphenol-A, o-Diphenyl oder p-Diphenyl mit 2 bis 6 Ethoxy- oder Propoxy-Gruppen sowie $C_2$-$C_{18}$-Alkandiole, die 1 bis 4 O- oder S-Atome in der Kohlenstoffkette enthalten können. Besonders bevorzugte Diole sind ethoxyliertes- oder propoxyliertes Bisphenol-A mit 2, 3 oder 4 Ethoxy- oder Propoxygruppen, Hexan-1,6-diol, Octan-1,8-diol, Nonan-1,9-diol, Decan-1,10-diol oder Dodecan-1,12-diol, Tetra- oder Pentaethylenglycol. Ganz besonders bevorzugt sind ethoxyliertes- oder propoxyliertes Bisphenol-A mit 2 oder 3 Ethoxy- oder Propoxygruppen, Decan-, Undecan- oder Dodecandiol sowie cyclische oder polycyclische aliphatische Diole, insbesondere Cyclohexandiol, Norbornandiol, Tricyclodecandiol und Tricyclodecandimethanol (Octahydro-4,7-methano-1H-indendimethanol).

Bevorzugte Diisocyanate sind Hexamethylen-1,6-diisocyanat (HMDI), 2,2,4-Trimethyl-hexamethylen-1,6-diisocyanat (TMDI), 1-Isocyanato-3-isocyanatomethyl-3,5,5-trimethylcyclohexan (Isophorondiisocyanat, IPDI), m-Tetramethylxylylendiisocyanat, (1,3-Bis(2-isocyanato-2-ppropyl)benzol, TMXDI), Toluol-2,4-diisocyanat (TDI), Diphenylmethan-4,4'-diisocyanat (MDI) und 1-Isocyanato-4-[(4-isocyanatocyclohexyl)methyl]cyclohexan ($H_{12}$MDI), wobei IPDI besonders bevorzugt ist.

Besonders bevorzugt sind Telechele gemäß der allgemeinen Formel (II)

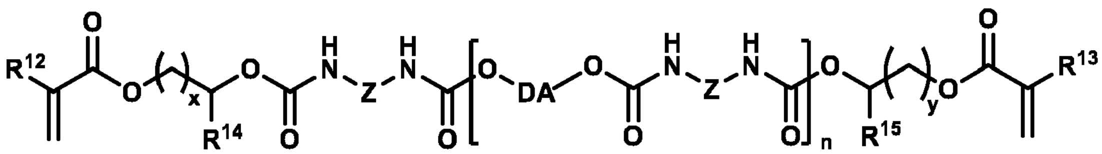

Formel (II)

in der die Variablen die folgenden Bedeutungen haben:

- $R^{12}$, $R^{13}$ unabhängig voneinander jeweils H oder Methyl, vorzugsweise Methyl,
- $R^{14}$, $R^{14}$ unabhängig voneinander jeweils H oder Methyl, vorzugsweise Methyl,
- x, y unabhängig voneinander jeweils eine ganze Zahl von 1 bis 11, vorzugsweise 1 bis 5,
- n 1, 2 oder 3, vorzugsweise 1,
- Z

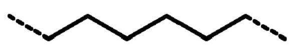
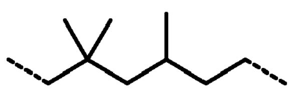
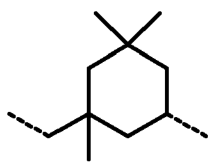
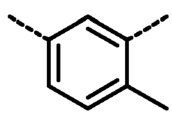
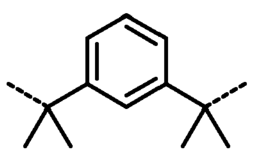
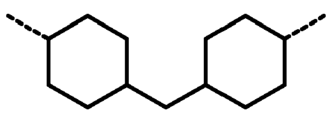
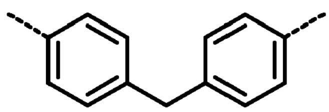

vorzugsweise:

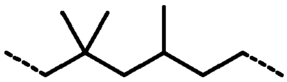

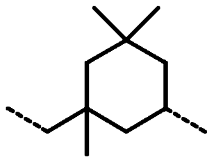

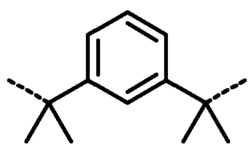

DA ein Strukturelement, das sich aus den Diolen HO-DA-OH durch Abspaltung der Wasserstoffatome der beiden Hydroxylgruppen ableitet.

Die Urethandi(meth)acrylat-Telechele tragen zwei radikalisch polymerisierbare (Meth)acrylatgruppen und eigenen sich als Vernetzer. Sie zeichnen sich durch eine gute radikalische Polymerisationsfähigkeit aus und ergeben aufgrund ihrer relativ hohen Molmasse Polymerisate mit einer geringeren Netzwerkdichte und geringem Polymerisationsschrumpf.

Telechele mit einer Molmasse von 1000 bis 3800 g/mol, die maximal 6 Urethangruppen enthalten, sind besonders bevorzugt. Die erfindungsgemäß bevorzugten Urethandi(meth)acrylat-Telechele zeichnen sich durch eine gute radikalische Polymerisationsfähigkeit aus. Außerdem verleihen sie den gehärteten Materialien gute kohäsive Eigenschaften.

Ganz besonders bevorzugte Urethandi(meth)acrylate sind UDMA (= 1,6-Bis-[2-methacryloyloxyethoxycarbonylamino]-2,4,4-trimethylhexan = Additionsprodukt von 1 mol 2,2,4-Trimethylhexamethylen-1,6-diisocyanat und 2 mol 2-Hydroxyethylmethacrylat), V-380 (Additionsprodukt von von 1 mol α,α,α',α'-Tetramethyl-m-xylylendiisocyanat und einer Mischung von 1,4 mol 2-Hydroxyethylmethacrylat und 0.6 mol 2-Hydroxypropylmethacrylat), und V-818 (Additionsprodukt von 1 mol Tricyclo[5.2.1.$0^{2,6}$]decanedimethanol und 2 mol 2-Isocyanatoethylmethacrylat). UDMA weist einen Urethangruppengehalt von 4,235 mmol/g auf.

Die erfindungsgemäß bevorzugten urethangruppenhaltigen Monomere (b) zeichnen sich durch eine gute radikalische Polymerisationsfähigkeit aus. Außerdem verleihen sie den gehärteten Materialien gute kohäsive Eigenschaften.

Wenn nicht anders angegeben, handelt es sich hierin bei der Molmasse von Oligomeren und Polymeren um die zahlenmittlere Molmasse $M_n$, die mittels Gelpermeationschromatographie (GPC) bestimmt wird.

Die Gelpermeationschromatographie (GPC) ist eine Relativmethode bei der die Moleküle aufgrund ihrer Größe, genauer gesagt aufgrund ihres hydrodynamischen Volumens, getrennt werden. Die Bestimmung der absoluten Molmasse erfolgt durch Kalibrierung mit bekannten Standards. Als Kalibrierstandard werden vorzugsweise engverteile Polystyrolstandards eingesetzt. Diese sind kommerziell erhältlich. Als Trennmaterial werden Styrol-Divinylbenzol-Säulen und Tetrahydrofuran (THF) als Elutionsmittel verwendet. Styrol-Divinylbenzol-Säulen eignen sich für organische lösliche synthetische Polymere. Die Messung erfolgt mit verdünnten Lösungen (0,05 - 0,2 Gew.-%) der zu untersuchenden Polymere.

Alternativ kann die zahlenmittlere Molmasse mit den bekannten Methoden der Gefrierpunktserniedrigung (Kryoskopie), der Siedepunktserhöhung (Ebullioskopie) oder aus der Erniedrigung des Dampfdrucks (Dampfdruckosmometrie) bestimmt werden. Hierbei handelt es sich um absolute Methoden, die keine Kalibrierstandards erfordern. Es werden Konzentrationsreihen von 4 bis 6 verdünnten Polymerlösungen mit Konzentrationen von 0,005 bis 0,10 mol/kg untersucht und dann die gemessenen Werte auf eine Konzentration von 0 mol/kg extrapoliert.

Die erfindungsgemäßen Zusammensetzungen können bis zu 60 Gew.-% an **Mono(meth)acrylaten (c)** enthalten, wobei das oder die Mono(meth)acrylate vorzugsweise aus aliphatischen, cycloaliphatischen, aromatischen, bicyclischen und tricyclischen Mono(meth)acrylate ausgewählt sind und die Gesamtmenge an Mono(meth)acrylaten die genannten Mengenbereiche nicht überschreitet. Besonders bevorzugte sind aliphatische Mono(meth)acrylate oder Mono(meth)acrylate mit einer cycloaliphatischen, aromatischen, bicyclischen oder tricyclischen Gruppe, wie z.B. Benzyl-, Tetrahydrofurfuryl- oder Isobornyl(meth)acrylat, 2-[(Methoxycarbonyl)amino]ethylmethacrylat, 2-[(Propoxycarbonyl) amino]-ethylmethacrylat, 2-[(Isopropoxycarbonyl)amino]-ethylmethacrylat, 2-[(Butoxycarbonyl)amino]ethylmethacrylat, 2-[(Hexyloxycarbonyl)-amino]-ethylmethacrylat, 2-[(Cyclohexyloxycarbonyl)-amino]-ethylmethacrylat, 2-[(Ethylcarbamoyl)oxy]-ethylmethacrylat, 2-[(Propylcarbamoyl)oxy]ethylmethacrylat, 2-[(Isopropylcarbamoyl)-oxy]ethylmethacrylat, 2-[(Hexylcarbamoyl)oxy]ethylmethacrylat, 2-[(2-Tetrahydrofurfuryloxycarbonyl)amino]ethylmethacrylat, 2-(2-Oxo-[1,3]-dioxolan-4-ylmethoxycarbonylamino)ethylmethacrylat, Methacrylsäure-2-(furan-2-yl-methoxycarbonylamino)ethylester, (1,3-Dioxolanon-2-on-4-yl)methylmethacrylat, 2-Phenoxyethyl(meth)acrylat, 2-(o-Biphenyloxy) ethyl(meth)acrylat, 2-Hydroxy-3-phenoxypropyl(meth)acrylat, 2-[(Benzyloxycarbonyl)amino]ethyl(meth)acrylat, 2-[(Benzylcarbamoyl)oxy]ethyl(meth)acrylat, 1-Phenoxypropan-2-yl-(meth)acrylat und 2-(p-Cumylphenoxy) ethyl(meth)acrylat, Tricylodecan-(meth)acrylat, Tricyclodecanmethyl(meth)-acrylat und 4,7,7-Trimethylbicyclo[2.2.1] heptanyl(meth)acrylat.

Erfindungsgemäß sind schwerflüchtige Mono(meth)acrylate (c) bevorzugt. Unter schwerflüchtigen Stoffen werden Verbindungen mit einer Verdunstungszahl von grö-ßer 35 verstanden. Die Verdunstungszahl (VD) gibt an,

wie schnell ein Stoff bei Raumtemperatur verdunstet. Sie wird nach der DIN 53170 bestimmt. Dabei wird die Zeit, in der ein Stoff komplett verdunstet (Verdunstungszeit = VDZ) mit der Zeit in Relation gesetzt, die Diethylether zum Verdunsten benötigt. Je höher die Verdunstungszahl ist, desto langsamer verdunstet ein Stoff.

Ganz besonders bevorzugte monofunktionelle Monomere sind Tetrahydrofurfuryl- und Isobornyl(meth)acrylat, 2-[(Benzyloxycarbonyl)-amino]-ethylmethacrylat, 2-[(Ethylcarbamoyl)-oxy]-ethylmethacrylat, 2-[(Benzylcarbamoyl)-oxy]-ethylmethacrylat, Methacrylsäure-2-(furan-2-yl-methoxycarbonylamino)-ethylester, 2-Phenoxyethyl(meth)acrylat, 2-(o-Biphenyloxy)ethyl(meth)acrylat, 2-Hydroxy-3-phenoxypropyl-(meth)acrylat, 1-Phenoxypropan-2-yl-(meth)acrylat, 2-(p-Cumylphenoxy)-ethyl-(meth)acrylat, Tricylodecan-(meth)acrylat, Tricyclodecanmethyl(meth)acrylat, 4,7,7-Trimethylbicyclo-[2.2.1]heptanyl(meth)acrylat und Mischungen davon.

Aliphatischen, cycloaliphatischen, aromatischen, bicyclischen und tricyclische Mono(meth)acrylate (c) zeichnen sich durch eine gute radikalische Polymerisationsfähigkeit aus. Darüber hinaus haben die Polymerisate dieser Mono(meth)acrylate einen vergleichsweise geringen Polymerisationsschrumpf und gute mechanische Eigenschaften. Aufgrund ihrer relativ hohen Molmasse (150 bis 350 g/mol) und ihrer relativ unpolaren Struktur haben die erfindungsgemäß bevorzugten Mono(meth)acrylate außerdem eine geringe Flüchtigkeit und eine vergleichsweise geringe Viskosität. Allerdings führen Mono(meth)acrylate zu einer Verringerung der Netzwerkdichte. Der Anteil an Mono(meth) acrylaten ist daher auf maximal 60 Gew.-%, vorzugsweise maximal 55 Gew.-% und besonders bevorzugt maximal 50 Gew.-% beschränkt. Ganz besonders bevorzugt sind Zusammensetzungen, die maximal 5 Gew.-% und am meisten bevorzugt keine monofunktionellen Monomere enthalten.

Die erfindungsgemäßen Zusammensetzungen können als **Komponente (d)** 0 bis 50 Gew.-% und vorzugsweise 0 bis 40 Gew-% eines oder mehrerer Di(meth)acrylate ohne Urethangruppen enthalten. Bevorzugte Di(meth)acrylate (d) sind Bisphenol-A-di(meth)acrylat, Bis-GMA (ein Additionsprodukt aus (Meth)acrylsäure und Bisphenol-A-diglycidylether), ethoxy- oder propoxyliertes Bisphenol-A-di(meth)acrylat A mit im Mittel 2, 3 oder 10 Ethoxy- oder Propoxygruppen, wie z.B. ein Bisphenol-A-di(meth)acrylat mit 3 Ethoxygruppen, oder 2,2-Bis[4-(2-(meth)acryloyloxypropoxy)phenyl] propan, Di-, Tri- oder Tetraethylenglycoldi(meth)acrylat, Trimethylolpropantri(meth)acrylat, Pentaerythrittetra(meth)acrylat sowie Glycerindi- und -tri(meth)acrylat, 1,4-Butandioldi(meth)acrylat, 1,10-Decandioldi(meth)acrylat, Bis[(meth) acryloyloxymethyl]tricyclo-[5.2.1.0$^{2,6}$]decan, Polyethylenglycol- oder Polypropylenglycoldi(meth)acrylat, wie z.B. Polyethylenglycol-200- oder -400-di(meth)acrylat oder 1,12-Dodecandioldi(meth)acrylat. Bevorzugte Di(meth)acrylate (d) sind Bis-GMA, ethoxy- oder propoxyliertes Bisphenol-A-di(meth)acrylat A mit im Mittel 2 oder 3 Ethoxy- oder Propoxygruppen, Tri- oder Tetraethylenglycoldi(meth)acrylat, Glycerindi- und -tri(meth)acrylat, 1,4-Butandioldi(meth)acrylat, 1,10-Decandioldi(meth)acrylat, Bis[(meth)acryloyloxymethyl]tricyclo-[5.2.1.0$^{2,6}$]decan, 1,12-Dodecandioldi(meth)acrylat. Besonders bevorzugte Di(meth)acrylate (d) sind: Bis-GMA, ethoxy- oder propoxyliertes Bisphenol-A-di(meth)acrylat A mit im Mittel 2 oder 3 Ethoxy- oder Propoxygruppen, Triethylenglycoldi(meth)acrylat, 1,10-Decandioldi(meth)acrylat, Bis[(meth)acryloyloxymethyl]tricyclo-[5.2.1.0$^{2,6}$]decan, 1,12-Dodecandioldi(meth)acrylat.

Die bevorzugten Di(meth)acrylatmonomere (d) zeichnen sich durch ein relativ geringes Mol-Gewicht aus, wobei Di(meth)acrylate (d) mit einem Mol-Gewicht im Bereich von 200 bis 800 g/mol und insbesondere 220 bis 650 g/mol besonders bevorzugt sind. Wegen des, insbesondere im Vergleich zu den Urethandi(meth)acrylat-Telechelen (b), geringen Mol-Gewichts bewirken die Di(meth)acrylatmonomere (d) eine relativ starke Vernetzung der Polymerisate. Der Anteil weiterer Di(meth)acrylate (d) ist daher auf maximal 50 Gew.-%, vorzugsweise maximal 40 Gew.-% beschränkt. Weiterhin ist es bevorzugt, dass die erfindungsgemäßen Zusammensetzungen neben den Urethan(meth)acrylaten (b) und den Di(meth)acrylaten (d) maximal 2 Gew.-%, besonders bevorzugt maximal 1 Gew.-%, an weiteren polyfunktionellen Monomere enthalten, wie beispielsweise Tri-, Tetra- oder höherfunktionelle (Meth)acrylate. Gemäß einer besonders bevorzugten Ausführungsform enthalten die erfindungsgemäßen Dentalwerkstoffe als Vernetzer ausschließlich Urethandi(meth)acrylate (b).

Urethandi(meth)acrylate (b), monofunktionelle Methacrylate (c), und Di(meth)acrylatmonomere (d) werden vorzugsweise in einem solchen Mengenverhältnis eingesetzt, dass Polymere mit einer Netzwerkdichte von vc = 800 bis 16.000 mol/m$^3$, bevorzugt 2000 bis 10.000 mol/m$^3$ besonders bevorzugt 2500 bis 8000 mol/m$^3$ erhalten werden. Die Vernetzungsdichte wird erfindungsgemäß maßgeblich durch das Verhältnis von vernetzenden zu nicht vernetzenden Monomeren eingestellt. Erfindungsgemäß bevorzugt sind Dentalwerkstoffe, in denen der Molenbruch der vernetzenden Monomeren in einem Bereich von 0,30 bis 1,0, besonders bevorzugt 0,5 zu 0,95 liegt. Ganz besonders bevorzugt sind Zusammensetzungen, die maximal 5 Gew.-% oder keine monofunktionellen Monomere enthalten.

Zur Berechnung des Molenbruchs werden alle radikalisch polymerisierbaren Komponenten der erfindungsgemäßen Werkstoffe herangezogen, d.h. insbesondere die Komponenten (b), (c) und (d). Unter vernetzenden Monomeren werden alle radikalisch polymerisierbaren Komponenten verstanden, die zwei oder mehr radikalisch polymerisierbare Gruppen aufweisen, d.h. insbesondere die Komponenten (b) und (d). Vernetzende Monomere werden auch als polyfunktionelle Monomere bezeichnet. Monofunktionelle Monomere sind Monomere mit nur einer radikalisch polymerisierbaren Gruppe.

Die Netzwerkdichte entspricht der Zahl der Netzknoten (in mol) je Volumeneinheit und lässt sich durch dynamisch-mechanische Messungen aus dem Plateauwert des Speichermoduls G' im elastischen Bereich berechnen.

Die Glasübergangstemperatur Tg und die Netzwerkdichte vc werden mit einem Rheometer bestimmt, vorzugsweise einem Anton Paar Rheometer MCR301. Hierzu werden Speicher- und Verlustmodul eines Prüfkörpers (25 x 5 x 1 mm, längs eingespannt) zwischen 25 °C und 250 °C vermessen (Frequenz 1 Hz, Deformation 0,05 %, Heizrate 2K/min). Tg ergibt sich dabei als das Maximum des Verlustfaktors tan $\delta$ (Verhältnis von Verlustmodul zu Speichermodul). Die Netzwerkdichte wird nach der Formel vc = G'/(RT) berechnet, mit G' als dem Speichermodul bei der Temperatur Tg + 50 K, R als der allgemeinen Gaskonstante und T als der Temperatur bei Tg + 50 K in Kelvin.

Die erfindungsgemäßen Zusammensetzungen können 0 bis 12 Gew.-% und vorzugweise 0 bis 10 Gew.-% eines oder mehrerer **Blockcopolymere und/oder Kern-Schale-Polymere (e)** als Schlagzähmodifikatoren enthalten.

Unter Blockcopolymeren werden Makromoleküle verstanden, die aus zwei oder mehr kovalent miteinander verbundenen Homopolymerblöcken bestehen. Erfindungsgemäß bevorzugte Blockcopolymere sind AB-Diblock- und insbesondere ABA-Triblockcopolymere.

Der A-Block ist ein Polymerisat, vorzugsweise ein Oligomer, das aus einem oder mehreren der folgenden Monomere aufgebaut ist: cyclische, aliphatische Ester oder Ether, Arylenoxid, Alkylenoxid, radikalisch polymerisierbare Monomere, beispielsweise $\alpha,\beta$-ungesättigte Säuren und $\alpha,\beta$-ungesättigte Säureester. Vorzugsweise ist der Block A ein Poly(meth)acrylat-, Polylacton-, Polyphenylenoxid- oder Polyalkylenoxid-Oligomer. Ganz besonders bevorzugt ist der Block A ein Polymerisat von Caprolacton, 2,6-Dialkyl-1,4-phenylenoxid und insbesondere von 2,6-Dimethyl-1,4-phenylenoxid, Ethylenoxid, Propylenoxid oder (Meth)acrylaten. Der A-Block ist damit vorzugsweise ein Polycaprolacton- (PCL), Poly(2,6-dimethyl-1,4-phenylenoxid)-, Poly(ethylenoxid)-, Poly(propylenoxid)- oder Poly(meth)acrylat-Oligomer.

Der B-Block ist vorzugsweise ein Polysiloxan- und/oder ein Polyvinyl- und/oder ein Polyalken- und/oder ein Polydien-Oligomer bzw. ein hydriertes Polydien-Oligomer. Besonders bevorzugt ist der B-Block ein Polydien-Oligomer bzw. ein hydriertes Polydien-Oligomer, Polyvinylalkanoat-Oligomer oder ein Polysiloxan-Oligomer gemäß der Formel $-O-(SiR^{16}{}_2-O)_P-$, in der

$R^{16}$ eine lineare $C_1$-$C_{20}$-Alkyl-, verzweigte $C_3$-$C_{12}$-Alkyl- oder $C_6$-$C_{20}$-Arylgruppe ist, wobei die einzelnen $R^{16}$-Reste gleich oder verschieden sein können, und
p eine Zahl von 3 bis 100, bevorzugt eine Zahl von 10 bis 50 ist.

Ganz besonders bevorzugt ist der B-Block ein Polymerisat von Dimethylchlorsilan, Cyclotri- oder Cyclotetradimethoxysilan, Isopren, Vinylacetat, Isobuten, cis-Butadien oder Ethylen. Der B-Block ist damit vorzugsweise ein Poly(dimethylsiloxan)- (PDMS), hydriertes Poly(isopren)-, Poly(vinylacetat)-, hydriertes Poly(isobuten)-, hydriertes cis-Poly(butadien)- oder Poly- (ethylen)-Oligomer.

Die B-Blöcke zeichnen sich durch eine relativ hohe Flexibilität aus. Unter flexiblen Blöcken werden Blöcke verstanden, die aus Monomeren gebildet werden, deren Homopolymeren eine Glasübergangstemperatur $T_G$ unter 50 °C, vorzugsweise unter 0 °C und ganz besonders bevorzugt im Bereich von -30 bis -110 °C haben. Blockcopolymere mit flexiblen Blöcken verbessern die Bruchzähigkeit, beinträchtigen aber die Biegefestigkeit und das E-Modul der Polymerisate deutlich weniger als innere Weichmacher.

Bevorzugt sind ABA- und AB-Blockcopolymere bei denen der oder die A-Blöcke vorzugsweise jeweils ein oligomerer Polycaprolacton-, Poly(2,6-dimethyl-1,4-phenylenoxid)-, Poly(ethylenoxid)-, Poly(propylenoxid)-, Poly(meth) acrylat- oder Poly(meth)acrylat-Styrol-Copolymer-Baustein sind und der B-Block vorzugsweise ein flexibler Poly(dimethylsiloxan)-, Poly(isopren)- oder hydriertes Poly(isopren)-, Poly(vinylacetat)-, Poly(isobuten)-, cis-Poly(butadien)- oder hydriertes cis-Poly(butadien)- oder ein Poly(ethylen)-Baustein ist.

Erfindungsgemäß besonders bevorzugt sind ABA-Blockcopolymere in denen die A-Blöcke jeweils ein oligomerer Polycaprolacton-, Poly(meth)acrylat- oder Poly(meth)acrylat-Styrol-Copolymer-Baustein sind und der B-Block ein flexibler Poly(dimethylsiloxan)-, hydriertes Poly(isopren)- oder hydriertes cis-Poly(butadien)-Baustein ist.

Ganz besonders bevorzugt sind Polyester-Polysiloxan-Blockcopolymere gemäß der folgenden allgemeinen Formel:

$$(PCL)_q\text{-b-}(PDMS)_r\text{-b-}(PCL)_q$$

in der

q jeweils eine Zahl von 5 bis 40, bevorzugt 10 bis 20, ist und
r eine Zahl von 10 bis 100, bevorzugt 30 bis 60, ist.

$(PCL)_q$ steht für Polycaprolacton, das aus q Caprolactonmonomeren aufgebaut ist, und (PDMS)r für Poly(dimethylsiloxan), das aus r Dimethylsiloxanmonomeren aufgebaut ist. Der Buchstabe b steht für *block.*

Weiter bevorzugt sind Poly(meth)acrylat-Polysiloxan-Blockcopolymere, die als A-Block einen Polymethylmethacrylat-Rest und als B-Block einen Polysiloxan-Rest enthalten, wobei der Polysiloxan-Rest vorzugsweise wie oben

definiert und ganz besonders bevorzugt ein Poly(dimethylsiloxan)-Rest ist.

Besonders bevorzugt sind außerdem die ABA-Triblockcopolymere PCL-b-PDMS-b-PCL und PMMA-b-PDMS-b-PMMA mit einem Molverhältnis A : B von 0,1 bis 5 und mit einer Molmasse von bevorzugt 3 bis 25 kDa, besonders bevorzugt 4 bis 20 kDa und ganz besonders bevorzugt 5 bis 10 kDa. Ein bevorzugtes Blockcopolymer ist PCL-b-PDMS-b-PCL, wobei die PDMS-Blöcke eine Molmasse von ca. 3200 g/mol und die PCL-Blöcke eine Molmasse von jeweils ca. 1600 g/mol aufweisen. PCL steht für Polycaprolacton, PDMS für Poly(dimethylsiloxan) und PMMA für Polymethylmethacrylat.

Blockcopolymere lassen sich mit den bekannten Methoden der lebenden bzw. kontrollierten Polymerisation herstellen, z.B. durch radikalische oder ionische (anionische und kationische) Polymerisation und auch durch metallkatalysierter Ringöffnungspolymerisation von cyclischen Monomeren, wie z.B. Caprolacton, wobei die kontrollierte radikalische Polymerisation, die lebende anionische Polymerisation und Ringöffnungspolymerisation bevorzugt sind. Blockcopolymere können aber auch durch die Verknüpfung von Endgruppen von Homopolymeren erhalten werden. Die erfindungsgemäß verwendeten Blockcopolymere können als Di- und Triblockcopolymere vorliegen.

AB-Blockcopolymere lassen sich beispielsweise herstellen, indem ein A-Block mit endständiger OH-Gruppe durch Veresterung mit einem B-Block verknüpft wird, der eine COOH-Gruppe aufweist. Endgruppenfunktionalisierte Homopolymerblöcke lassen sich relativ einfach mit den Methoden der geregelten radikalischen Polymerisation oder durch Endcapping bei der anionischen Polymerisation herstellen. Beispielsweise wird das Monomer A anionisch polymerisiert und durch Endcapping eine OH-Gruppe eingeführt. Die OH-Endgruppe lässt sich dann z.B. mit α-Bromisobuttersäure verestern. Die dabei erhaltene Brom-Endgruppe fungiert dann als Startzentrum für die Bildung des B-Blocks durch ATRP (Atom Transfer Radical Polymerization) von Monomer B, initiiert durch Metallkomplexe beispielsweise von Cu(I), Ru(I) oder Fe(II).

Triblockcopolymere lassen sich auf analoge Weise herstellen. Beispielsweise wird durch anionische Polymerisation von Monomer B über einen Dianionen-Mechanismus ein B-Block hergestellt. Der gebildete B-Mittelblock trägt beidseitig jeweils eine Anion-Endgruppe, die die anionische Polymerisation von Monomer A unter Bildung der beiden A-Blocke initiiert (Methode 1). Die Veresterung eines telechelen B-Blocks, der an beiden Enden jeweils eine geeignete funktionelle Gruppe, z.B. eine OH-Gruppe trägt, mit zwei A-Blöcken, die nur einseitig z.B. mit einer COOH-Gruppe, funktionalisiert sind, ergibt ABA-Triblockcopolymere (Methode 2). Schließlich lassen sich OH-telechele Homopolymere von Monomer B mit α-Bromisobuttersäure verestern. Die beiden so gebildeten Brom-Endgruppen im Homopolymerblock B lassen sich dann als Startzentrum für die Bildung der beiden A-Blöcke durch ATRP nutzen (Methode 3).

Bei der Synthese der Blockcopolymeren lassen sich auch end- oder seitenständig polymerisationsfähige (Meth)acrylatgruppen einführen. Diese bewirken eine bessere Einbindung der Blockcopolymere in die gebildeten Polymernetzwerke durch radikalische Copolymerisation der (Meth)acrylatgruppen.

Die Monomere werden vorzugsweise so gewählt, dass die A-Blocke mit der Harzmatrix, d.h. der Mischung der Bestandteile (a) bis (d), mischbar und der B-Block nicht mit der Harzmatrix mischbar ist. Die Mischbarkeit wird hier im Sinne der Thermodynamik in Bezug auf die Einphasigkeit verstanden. Danach wird unter einem mischbaren Polymerblock ein Polymerblock aus einem Monomer verstanden, dessen Homopolymer in der Harz-Matrix löslich ist, so dass die Mischung eine Transparenz von mindestens 95 % aufweist. Ist die Mischung dagegen trüb oder opak, d.h. ist die Transparenz deutlich kleiner als 95 %, so ist das Homopolymer und damit der entsprechende Polymerblock nicht mit der Harz-Matrix mischbar. Die Transparenz wird mit einem Spektrophotometer an 1 mm dicken, auf Hochglanz polierten Prüfkörpern in Transmission (D65) gemäß der Norm ISO 10526:1999 gemessen, z.B. mit einem Konika-Minolta Spektrophotometer des Typs CM-5.

Die erfindungsgemäßen Zusammensetzungen können als Bruchzähigkeitsmodifikator alternativ oder vorzugsweise zusätzlich zu den Blockcopolymeren ein oder mehrere Polymerpartikel mit Kern-Schale-Struktur enthalten. Bevorzugt sind Polymerpartikel, die eine weichen Kern, vorzugsweise aus Polybutadien, hydriertem Polybutadien, Polyisopren, hydriertem Polyisopren, Polybutylacrylat, MMA-Butadien-Styrol-Copolymeren (MBS) oder Polydimethylsiloxan, und eine harte Schale aufweisen, vorzugsweise aus PMMA oder einem MMA-Styrol-Copolymer.

Unter weichen oder flexiblen Polymeren werden Polymere mit einer Glasübergangstemperatur $T_G$ unter 50 °C, vorzugsweise unter 0°C und ganz besonders bevorzugt im Bereich von -30 bis -110°C verstanden. Ein bevorzugtes konkretes Beispiel ist PDMS mit einer $T_G$ von ca. -110°C. Unter harten Polymeren werden Polymere mit einer Glasübergangstemperatur über 50°C und bevorzugt über 80 °C verstanden. Ein bevorzugtes konkretes Beispiel ist PMMA mit einer $T_G$ von 105-120 °C.

Die bruchzähigkeitsmodifizierende Wirkung der CSP-Partikeln in radikalischen Di(meth)acrylat-Polymernetzwerken hängt vor allem von der Art der CSP-Partikel, der Partikelgröße, der Vernetzungsdichte und dem Gewichtsverhältnis von Kern zu Schale ab, das vorzugweise in einem Bereich von 1:1 bis 200:1 liegt. Die Vernetzungsdichte wird maßgeblich durch den Anteil vernetzender Monomere im Partikelkern bestimmt.

Dieser liegt vorzugsweise in einem Bereich von 1 bis 10 Gew.-%, bezogen auf die Masse des Kerns. Erfindungsgemäß sind Partikel mit einer Teilchengröße von 0,20 bis 50,0 µm bevorzugt. Bei der Einarbeitung der CSP-Partikel in den Dentalwerkstoff ist auf eine gute Dispergierung zu achten.

Der oder die optionalen Bruchzähigkeitsmodifikatoren werden bei Bedarf vorzugsweise in einer Menge von 1 bis

10 Gew.-%, besonders bevorzugt in einer Menge von 2 bis 8 Gew.-% und ganz besonders bevorzugt 3 bis 6 Gew.-% eingesetzt, bezogen auf das Gesamtgewicht des Dentalwerkstoffs.

Kern-Schale-Polymere haben den Nachteil, dass sie die Transparenz der Zusammensetzungen beeinträchtigen können, was sich bei der Photopolymerisation negativ auf die Durchhärtungstiefe und bei dentalen Formkörpern auch auf die Ästhetik auswirken kann. Erfindungsgemäß sind daher Werkstoffe bevorzugt, die maximal 3 Gew.-% und besonders bevorzugt keine Kern- Schale-Partikel enthalten.

Die erfindungsgemäßen Zusammensetzungen enthalten mindestens einen **phenolischen Inhibitor (f).** Unter Inhibitoren werden radikalfangende Stoffe verstanden, die mit Radikalen neue, stabile Radikale bilden, die nicht mehr weiterreagieren. Inhibitoren werden auch als Polymerisationsinhibitoren bezeichnet und zur Verbesserung der Lagerstabilität radikalisch polymerisierbarer Materialien eingesetzt. Es wurde überraschenderweise gefunden, dass phenolischen Inhibitoren in einer Menge von 300 bis 1200 ppm nicht nur die Lagerstabilität von radikalisch polymerisierbaren Zusammensetzungen verbessern, sondern auch Verfärbungen verhindern können, die durch urethangruppenhaltige Monomere und Photoinitiatoren verursacht werden, insbesondere in Kombination mit Photoinitiatoren gemäß der Formel (I). Phenolische Inhibitoren, sind Phenolderivate, die einen Benzolring enthalten, der mit mindestens einer freien OH-Gruppe substituiert ist.

Bevorzugte Inhibitoren sind Hydrochinonmonomethylether (MEHQ, CAS: 150-76-5), 2,6-Di-tert.-butyl-4-methyl-phenol (BHT, CAS: 128-37-0), 2,6-Di-tert.-butylphenol (CAS: 128-39-2), Pyrogallol (CAS: 87-66-1), Hydrochinon (CAS: 123-31-9), 2-tert.-Butylhydrochinon (CAS: 1948-33-0), 4-tert.-Butylbrenzcatechin (CAS: 98-29-3) oder 6-tert.-Butyl-2,4-xylenol (CAS: 1879-09-0), wobei MEHQ, Pyrogallol und BHT besonders bevorzugt sind.

Die erfindungsgemäßen Photoinitiatorsysteme ermöglichen die Herstellung von Polymerisaten, die direkt nach der Polymerisation oder einer kurzen Lagerzeit keine oder nur eine geringe Gelbfärbung aufweisen. Verfärbungen werden durch einen Stresstest ermittelt, bei dem das gehärtete Polymerisat in Wasser bei 50°C gelagert wird. Die Farbbestimmung erfolgt gemäß dem CIE-Lab-Farbsystem. CIE steht für International Commission on Illumination. Dabei wird die Farbe im Farbraum durch 3 Koordinaten, die rechtwinklig aufeinander stehen, beschrieben: Die Helligkeit L*, die von 0 (reines Schwarz) bis 100 (reines Weiß) reicht. a* stellt die rot-grün-Achse dar, wobei negative Werte grün und positive Werte rot sind. b* stellt die gelb-blau-Achse dar, wobei negative Werte blau und positive Werte gelb sind. Farbunterschiede, der Farbabstand, können mit dem sog. Euklidischen Abstand Delta E* ($\Delta$E*) bewertet werden, wobei E für Empfindung steht. Für die Bewertung von Verfärbungen dentaler Werkstoffe ist insbesondere die Gelbfärbung und damit der b*-Wertes von praktischer Relevanz.

Die erfindungsgemäßen Werkstoffe zeichnen sich dadurch aus, dass sie nach der Polymerisation einen b*-Wert < 4,5, vorzugsweise < 4 und besonders bevorzugt < 3 aufweisen. Dieser Wert wird direkt nach der Polymerisation oder nach nur kurzer Lagerung erreicht, vorzugsweise nach maximal 200 h. Dies bedeutet, dass polymerisationsbedingte Verfärbungen innerhalb von 200 h weitestgehend abgebaut werden. Der zeitliche Verlauf der Abnahme der Gelbverfärbung lässt sich durch den kinetischen Entfärbungsvektor KDV (kinetic decoloration vector) beschreiben, der auf die in den Ausführungsbeispielen beschriebene Weise ermittelt wird. Die erfindungsgemäßen Zusammensetzungen weisen einen kinetischen Entfärbungsvektor von kleiner 3,5, besonders bevorzugt kleiner 3,0 und ganz besonders bevorzugt kleiner 2,5 auf.

Die erfindungsgemäßen Initiatorsysteme ermöglichen zudem die Herstellung radikalisch polymerisierbarer Werkstoffe mit guter Photopolymerisationsreaktivität und guten mechanischen Eigenschaften nach der Härtung. Die Werkstoffe haben nach der Photopolymerisation vorzugsweise eine Biegefestigkeit von größer 40 MPa, vorzugsweise 40 MPa bis 400 MPa, besonders bevorzugt 60 MPa bis 400 MPa, und einen Biegemodul von größer 1,5 GPa, vorzugsweise 1,5 GPa bis 6 GPa, gemessen nach ISO 20795-1. Die Werkstoffe sind farb- und lagerstabil und weisen eine hohe Polymerisationsgeschwindigkeit sowie einen hohen Doppelbindungsumsatz auf.

Die erfindungsgemäßen Zusammensetzungen können einen oder mehrere **Füllstoffe (g)** enthalten, vorzugsweise partikuläre oder faserförmige anorganische oder organische Füllstoffe oder Kompositfüllstoffe. Füllstoffe ermöglichen eine Beeinflussung der mechanischen Eigenschaften. Besonders bevorzugt sind anorganische partikuläre Füllstoffe.

Bevorzugte anorganische Füllstoffe sind Oxide, wie $SiO_2$, $ZrO_2$ und $TiO_2$ oder Mischoxide aus $SiO_2$, $ZrO_2$, ZnO und/oder $TiO_2$, nanopartikuläre oder mikrofeine Füllstoffe, wie pyrogene Kieselsäure oder Fällungskieselsäure, Glaspulver, wie Quarz-, Glaskeramik-, Borosilikat- oder röntgenopake Glaspulver, vorzugsweise Barium- oder Strontiumaluminiumsilikatgläser, und röntgenopake Füllstoffe, wie Ytterbiumtrifluorid, Tantal(V)-oxid, Bariumsulfat oder Mischoxide von $SiO_2$ mit Ytterbium(III)-oxid oder Tantal(V)-oxid. Weiterhin können die erfindungsgemäßen Dentalwerkstoffe faserförmige Füllstoffe, Nanofasern, Whiskers oder Mischungen davon enthalten. Gemäß einer bevorzugten Ausführungsform enthalten die erfindungsgemäßen Werkstoffe keine Fluoroaluminosilikatgläser, Calciumaluminiumsilikatgläser oder andere Füllstoffe, die mit organischen Säuren im Sinne einer Säure-Base-Reaktion reagieren.

Bevorzugt weisen die Oxide eine Partikelgröße von 0,010 bis 15 $\mu$m, die nanopartikulären oder mikrofeinen Füllstoffe eine Partikelgröße von 10 bis 300 nm, die Glaspulver eine Partikelgröße von 0,10 bis 15 $\mu$m, vorzugweise von 0,2 bis 1,5 $\mu$m, und die röntgenopaken Füllstoffe eine Partikelgröße von 0,2 bis 5 $\mu$m auf.

Besonders bevorzugte Füllstoffe sind Mischoxide aus SiOz und ZrOz, mit einer Partikelgröße von 10 bis 300 nm, Glaspulver mit einer Partikelgröße von 0,2 bis 1,5 µm, insbesondere röntgenopake Glaspulver z.B. von Barium- oder Strontiumaluminiumsilikatgläsern, und röntgenopake Füllstoffe mit einer Partikelgröße von 0,2 bis 5 µm, insbesondere Ytterbiumtrifluorid und/oder Mischoxide von $SiO_2$ mit Ytterbium(III)- oxid.

Zur Verbesserung des Verbundes zwischen den Füllstoffpartikeln und der vernetzten Polymerisationsmatrix können $SiO_2$-basierende Füllstoffe mit methacrylatfunktionalisierten Silanen oberflächenmodifiziert werden. Ein bevorzugtes Beispiel für solche Silane ist 3-Methacryloyloxypropyltrimethoxysilan. Zur Oberflächenmodifizierung von nichtsilikatischen Füllstoffen wie ZrOz oder TiOz können auch funktionalisierte saure Phosphate, wie z.B. 10-Methacryloyloxydecyldihydrogenphosphat eingesetzt werden.

Weitere bevorzugte Füllstoffe sind partikuläre Wachse, insbesondere Carnaubawachs, vorzugsweise mit eine Partikelgröße von 1 bis 10 µm, unvernetzte oder teilvernetzte Polymethylmethacrylat (PMMA)-Partikel, vorzugsweise mit einer Partikelgröße von 500 nm bis 10 µm, sowie Polyamid-12 Partikel, vorzugsweise mit einer Partikelgröße von 5 bis 10 µm.

Außerdem können die erfindungsgemäßen Dentalwerkstoffe einen sog. Präpolymerfüllstoff oder Isofüllstoff enthalten, d.h. ein gemahlenes Komposit, das vorzugsweise eine breite Partikelgrößenverteilung z. B. mit Partikelgrößen von 0,05 10 bis 20 µm, insbesondere etwa 0,1 bis etwa 10 µm, aufweist. Vorzugsweise ist der Präpolymerfüllstoff oder Isofüllstoff oberflächenmodifiziert, insbesondere silanisiert.

Wenn nicht anders angegeben, handelt es sich bei allen Partikelgrößen hierin um gewichtsmittlere Partikelgrößen, wobei die Partikelgrößenbestimmung im Bereich von 0,1 µm bis 1000 µm mittels statischer Lichtstreuung erfolgt, vorzugsweise mit einem statischen Laserstreuungs-Partikelgrößen-Analysator LA-960 (Horiba, Japan). Hierbei werden als Lichtquellen eine Laser-Diode mit einer Wellenlänge von 655 nm und eine LED mit einer Wellenlänge von 405 nm verwendet. Der Einsatz von zwei Lichtquellen mit unterschiedlichen Wellenlängen ermöglicht die Vermessung der gesamten Partikelgrößenverteilung einer Probe in nur einem Messungsdurchgang, wobei die Messung als Nassmessung durchgeführt wird. Hierzu wird eine 0,1 bis 0,5%ige wässrige Dispersion des Füllstoffs hergestellt und deren Streulicht in einer Durchflusszelle gemessen. Die Streulichtanalyse zur Berechnung von Partikelgröße und Partikelgrößenverteilung erfolgt gemäß der Mie-Theorie nach DIN/ISO 13320.

Partikelgrößen kleiner als 0,1 µm werden vorzugsweise mittels Dynamischer Lichtstreuung (DLS) bestimmt. Die Messung der Partikelgröße im Bereich von 5 nm bis 0,1 µm erfolgt vorzugsweise durch Dynamische Lichtstreuung (DLS) von wässrigen Partikeldispersionen, vorzugsweise mit einem Malvern Zetasizer Nano ZS (Malvern Instruments, Malvern UK) mit einem He-Ne-Laser mit einer Wellenlänge von 633 nm, bei einem Streuwinkel von 90° bei 25°C

Die Lichtstreuung nimmt mit abnehmender Partikelgröße ab. Partikelgrößen kleiner als 0,1 µm können auch mittels REM- oder TEM-Spektroskopie bestimmt werden. Die Transmissionselektronenmikroskopie (TEM) wird vorzugsweise mit einem Philips CM30 TEM bei einer Beschleunigungsspannung von 300 kV durchgeführt. Für die Probenvorbereitung werden Tropfen der Partikeldispersion auf einem 50 A dickem Kupfergitter (Maschenweite 300 Mesh) aufgebracht, das mit Kohlenstoff beschichtet ist, und im Anschluss das Lösungsmittel verdampft.

Die Füllstoffe werden nach ihrer Partikelgröße unterteilt in Makrofüller und Mikrofüller, wobei Füllstoffe mit einer mittleren Partikelgröße von 0,2 bis 10 µm als Makrofüller und Füllstoffe mit einer mittlere Partikelgröße von ca. 5 bis 100 nm als Mikrofüller bezeichnet werden. Makrofüller werden z.B. durch Mahlen z.B. von Quarz, röntgenopaken Gläsern, Borosilikaten oder von Keramik gewonnen und bestehen meist aus splitterförmigen Teilen. Als Mikrofüller werden vorzugsweise pyrogenes $SiO_2$ oder Fällungskieselsäure eingesetzt, oder Mischoxide, z.B. $SiO_2$-$ZrO_2$, die durch hydrolytische Cokondensation von Metallalkoxiden zugänglich sind. Die Mikrofüller haben vorzugsweise eine mittlere Partikelgröße von ca. 5 bis 100 nm. Mikrofüller. Füllstoffe mit geringer Partikelgröße haben eine größere Verdickungswirkung.

In einer bevorzugten Ausführungsform enthalten die erfindungsgemäßen Dentalwerkstoffe eine Mischung von zwei oder mehreren Füllstoffen, insbesondere von zwei oder mehreren Füllstoffen mit unterschiedlichen Partikelgrößen. Es wurde gefunden, dass durch die Verwendung solcher Füllstoffmischungen die Viskosität der Werkstoffe nicht übermäßig erhöht wird und die Zusammensetzungen daher mit generativen Verfahren, wie z.B. mit Hilfe der Stereolithographie, gut verarbeitbar sind. Der Gesamtgehalt an Füllstoffen liegt vorzugsweise in einem Bereich von 0 bis 30 Gew.-%, besonders bevorzugt von 0 bis 20 Gew.-%.

Die erfindungsgemäßen Zusammensetzungen können darüber hinaus ein oder mehrere **Additive (h)** enthalten, insbesondere UV-Absorber, optische Aufheller, Farbmittel, Weichmacher und/oder Thixotropieadditive.

Die erfindungsgemäßen Zusammensetzungen können einen oder mehrere **UV-Absorber** enthalten. UV-Absorber dienen dazu, bei der lichtinduzierten Härtung der erfindungsgemäßen Zusammensetzung die Eindringtiefe des Lichts und damit die Polymerisationstiefe zu verringern. Dies erweist sich insbesondere bei stereolithographischen Anwendungen als vorteilhaft, da bei der Stereolithographie lediglich dünne Schichten gehärtet werden sollen. Durch Verwendung eines UV-Absorbers kann bei stereolithographischen Verfahren die Präzision verbessert werden.

Bevorzugt sind UV-Absorber auf der Basis von Benzotriazol, Benzophenon oder Triazine. Besonders bevorzugte UV-Absorber sind 2,2'-Methylenbis[6-(2H-benzotriazol-2-yl)-4-(1,1,3,3-tetramethylbutyl)phenol], 2,2',4,4'-Tetrahydroxybenzophenon, 2-tert-Butyl-6-(5-chloro-2H-benzotriazol-2-yl)-4-methylphenol (Bumetrizol), 2,2'-Benzol-1,4-diyl-

bis(4h-3,1-benzoxazin-4-on), 2-(4,6-Bis-(2,4-dimethylphenyl)-1,3,5-triazin-2-yl)-5-(octyloxy)-phenol, 2-(2-Hydroxy-5-methylphenyl)benzotriazol, 2-(2-Hydroxyphenyl)-benzotriazol, 2-(2H-Benzotriazol-2-yl)-6-dodecyl-4-methylphenol, 2-(2'-Hydroxy-3', 5'-di-t-butylphenyl)-5-chlorobenzotriazole, 2,2'-Dihydroxy-4-methoxybenzophenon und 2,2'-Dihydroxy-4,4'-dimethoxybenzophenon. Weiter bevorzugt sind sogenannte Hindered Amine Light Stabilizers wie Bis(1,2,2,6,6-pentamethyl-4-piperidyl)sebacat, Methyl-1,2,2,6,6-pentamethyl-4-piperidylsebacat, Bis(1-octyloxy-2,2,6,6-tetramethyl-4-piperidyl)sebacat und Bis(1,2, 2,6,6-pentamethyl-4-piperidyl)-[[3,5-bis(1,1-dimethylethyl)-4-hydroxyphenyl]methyl]butylmalonat. Ganz besonders bevorzugte UV-Absorber sind Bumetrizol und 2,2',4,4'-Tetrahydroxybenzophenon.

Der UV-Absorber weist vorzugsweise ein Absorptionsmaximum auf, das der Wellenlänge des zur Härtung eingesetzten Lichts entspricht. Vorteilhaft sind UV-Absorber mit einem Absorptionsmaximum im Bereich von 320 bis 500 nm und vorzugsweise 380 bis 480 nm, wobei UV-Absorber mit einem Absorptionsmaximum unterhalb von 400 nm besonders bevorzugt sind.

UV-Absorber werden ggf. in einer Menge von vorzugsweise 0 bis 1,0 Gew.-%, besonders bevorzugt 0,01 bis 0,5 Gew.-% eingesetzt. Bumetrizol wird vorzugsweise in einer Menge von 0,01 bis 0,2 Gew.-%, besonders bevorzugt 0,02 bis 0,15 Gew.-%, und 2,2',4,4'-Tetrahydroxybenzophenon in einer Menge von 0,01 bis 0,07 Gew.-% verwendet. Alle Angaben beziehen sich auf das Gesamtgewicht des Werkstoffs. Dentalwerkstoffe, die keinen UV-Absorber enthalten, sind bevorzugt.

Die erfindungsgemäßen Zusammensetzungen können weiterhin einen oder mehrere **optische Aufheller** enthalten. Erfindungsgemäß bevorzugt sind optische Aufheller, die Licht im UV-Bereich absorbieren, d.h. Licht mit einer Wellenlänge unterhalb von 400 nm. Durch die Zugabe eines optischen Aufhellers kann die Eindringtiefe des Lichts und damit die Durchhärtungstiefe verringert und so die Präzision bei stereolithographischen Prozessen erhöht werden. Besonders bevorzugt sind optische Aufheller, die in der Lage sind, das im UV-Bereich absorbierte Licht als Licht mit einer Wellenlänge von 400 bis 450 nm wieder zu emittieren. Solche optischen Aufheller erhöhen die Reaktivität der Werkstoffe, indem sie das aufgenommene kurzwellige Licht aufgrund ihrer Fluoreszenz als längerwelliges Blaulicht abstrahlen und damit zusätzliche Lichtleistung zur Photoinitiierung bereitstellen. Erfindungsgemäß bevorzugte optische Aufheller sind 2,5-Bis(5-tert-butyl-benzoxazol-2-yl)thiophen und Fluoreszenzmittel in Form von Terephthalsäurederivaten, wie z.B. 2,5-Dihydroxyterephthalsäurediethylester oder Diethyl-2,5-dihydroxyterephthalat.

Der oder die optischen Aufheller werden ggf. in einer Menge von vorzugsweise 0 bis 0,1 Gew.-%, besonders bevorzugt 0,001 bis 0,05 Gew.-% und ganz besonders bevorzugt 0,002 bis 0,02 Gew.-% eingesetzt, jeweils bezogen auf das Gesamtgewicht des Werkstoffs. Dentalwerkstoffe, die keinen optischen Aufheller enthalten, sind bevorzugt.

Optische Aufheller können in Kombination mit UV-Absorbern eingesetzt werden. In diesem Fall ist es bevorzugt, dass das Gewichtsverhältnis von UV-Absorber zu optischem Aufheller in einem Bereich von 2:1 bis 50:1, besonders bevorzugt 2:1 bis 30:1 und ganz besonders bevorzugt 2:1 bis 5:1 oder 10:1 bis 25:1 liegt. Bevorzugt sind Kombinationen, die als UV-Absorber 2,2',4,4'-Tetrahydroxybenzophenon oder Bumetrizol und als optischen Aufheller 2,5-Bis(5-tert-butyl-benzoxazol-2-yl)thiophen enthalten. Ganz besonders bevorzugt ist die Kombination von 2,2',4,4'-Tetrahydroxybenzophenon und 2,5-Bis(5-tert-butyl-benzoxazol-2-yl)thiophen in einem Gewichtsverhältnis von 2:1 bis 10:1, vorzugsweise 2:1 bis 5:1, oder die Kombination von Bumetrizol und 2,5-Bis(5-tert-butyl-benzoxazol-2-yl)thiophen in einem Gewichtsverhältnis von 5:1 bis 30:1, vorzugsweise 10:1 bis 20:1.

Die erfindungsgemäßen Zusammensetzungen können außerdem **Farbmittel** enthalten, vorzugsweise in einer Konzentration von 0,0001 bis 0,5 Gew.-%. Die Farbmittel dienen primär ästhetischen Zwecken. Erfindungsgemäß bevorzugte Farbmittel sind organische Farbstoffe und Pigmente, insbesondere Azofarbstoffe, Carbonylfarbstoffe, Cyaninfarbstoffe, Azomethine und Methine, Phthalocyanine und Dioxazine. Besonders bevorzugt sind Farbstoffe, die in den erfindungsgemäßen Werkstoffen löslich sind, insbesondere Azofarbstoffe. Als Farbmittel eignen sich außerdem anorganische und insbesondere organische Pigmente, die sich gut in den erfindungsgemäßen Dentalwerkstoffe dispergieren lassen. Bevorzugte anorganische Pigmente sind Metalloxide oder Hydroxide, wie z.B. Titandioxid oder ZnO als Weißpigmente, Eisenoxid (FezOs) als Rotpigment oder Eisenhydroxid (FeOOH) als Gelbpigment. Bevorzugte organische Pigmente sind Azopigmente, wie z.B. Monoazogelb und Orangepigmente, Diazopigmente oder β-Naphthol-Pigmente, und Nichtazo- oder polycyclische Pigmente, wie z.B. Phthalocyanin-, Chinacridon-, Perylen- und Flavanthron-Pigmente. Besonders bevorzugt sind Azopigmente und Nichtazopigmente.

Außerdem können die erfindungsgemäßen Zusammensetzungen einen oder mehrere **Weichmacher** enthalten. Weichmacher verhindern, dass die Polymerisate nach der photochemischen Aushärtung und einem evtl. Trocknen brüchig werden. Darüber hinaus sorgen Weichmacher für eine ausreichende Flexibilität. Weichmacher werden vorzugsweise in einer Konzentration von 0,2 bis 5 Gew.-% zugesetzt. Bevorzugte Weichmacher sind Phthalate, wie z.B. Dibutyl- oder Dihexylphthalat, nichtsaure Phosphate, wie z.B. Tributyl- oder Trikresylphosphat, n-Octanol, Glycerin oder Polyethylenglykole. Besonders bevorzugt sind Weinsäure- oder Citronsäureester, wie z.B. Citronensäuretriester, die sich durch eine gute Biokompatibilität auszeichnen.

Die erfindungsgemäßen Zusammensetzungen können ein oder mehrere **Thixotropieadditive** enthalten. Diese Additive bewirken eine Verdickung der Werkstoffe und können so beispielsweise ein Sedimentieren der Füllstoffe verhindern. Insbesondere füllstoffhaltige Werkstoffe enthalten daher vorzugweise mindestens ein Thixotropieadditiv.

Bevorzugte Thixotropieadditive sind OH-Gruppen-haltige Polymere, wie z.B. Cellulosederivate, und anorganische Stoffe, wie z.B. Schichtsilicate. Um die Viskosität der Werkstoffe nicht zu stark zu erhöhen, enthalten die erfindungsgemäßen Dentalwerkstoffe vorzugsweise nur 0 bis 3,0 Gew.-%, besonders bevorzugt 0 bis 2,0 Gew.-% und ganz besonders bevorzugt 0,1 bis 2,0 Gew.-% Thixotropieadditiv, bezogen auf das Gesamtgewicht des Werkstoffs.

Bestimmte Füllstoffe, wie z.B. hochdisperses $SiO_2$, d.h. SiOz mit geringer Primärteilchengröße (< 20 nm) und hoher spezifischer Oberfläche (> 100 $m^2/g$) haben ebenfalls eine thixotropierende Wirkung. Solche Füllstoffe können Thixotropieadditive ersetzen.

Die rheologischen Eigenschaften der erfindungsgemäßen Zusammensetzungen werden an den gewünschten Anwendungszweck angepasst. Materialen zur stereolithographischen Verarbeitung werden vorzugsweise so eingestellt, dass ihre Viskosität im Bereich von 50 mPa·s bis 100 Pa·s, bevorzugt 100 mPa·s bis 10 Pa·s, besonders bevorzugt 100 mPa·s bis 5 Pa·s liegt. Die Viskosität wird bei 25°C mit einem Kegel-Platte-Viskosimeter bestimmt (Scherrate 100/s). Besonders bevorzugt weisen die erfindungsgemäßen Dentalwerkstoffe eine Viskosität < 10 Pa·s und ganz besonders bevorzugt < 5 Pa·s bei 25°C auf. Die Viskosität wird vorzugsweise mit einem Anton Paar Viskosimeter des Typs MCR 302 mit CP25-2 Konus-Platte-Messmittel und einem Messspalt von 53 µm in Rotation bei einer Scherrate von 100/s bestimmt. Aufgrund der geringen Viskosität sind die erfindungsgemäßen Zusammensetzungen besonders dazu geeignet, mit Hilfe von generativen Fertigungsverfahren, wie z.B. 3D-Druck oder Stereolithographie, verarbeitet zu werden. Die Verarbeitungstemperatur liegt vorzugsweise in einem Bereich von 10 bis 70°C, besonders bevorzugt 20 bis 30°C.

Die erfindungsgemäßen Zusammensetzungen eignen sich besonders als Dentalwerkstoffe und insbesondere zur Herstellung oder Reparatur von dentalen Formteilen, wie z.B. Dentalrestaurationen, Prothesen, Prothesenwerkstoffen, künstlichen Zähnen, Inlays, Onlays, Kronen, Brücken, Bohrschablonen, Einprobekörpern oder orthodontischen Vorrichtungen. Die Verwendung der erfindungsgemäßen Zusammensetzungen als Dentalwerkstoffe ist ebenfalls Gegenstand der Erfindung.

Weiterhin betrifft die vorliegende Erfindung auch ein Verfahren zur Herstellung von dentalen Formteilen, insbesondere zur Herstellung der oben genannten dentalen Fortteile, bei dem eine erfindungsgemäße Zusammensetzung mit Hilfe von Licht gehärtet wird, um das dentale Formteil zu ergeben. Weiterhin betrifft die Erfindung auch dentale Formteile, insbesondere die oben genannten Formteile, die durch ein derartiges Verfahren erhältlich sind.

Die Herstellung oder Reparatur von dentalen Formteilen erfolgt vorzugsweise extraoral. Des Weiteren ist es bevorzugt, dass die Herstellung oder Reparatur von dentalen Formteilen durch ein generatives Verfahren, insbesondere mit Hilfe von 3D-Druck oder einem Lithographie-basierten Verfahren, wie z.B. Stereolithographie, erfolgt.

Die Herstellung von erfindungsgemäßen dentalen Formteilen erfolgt vorzugsweise durch ein stereolithographisches Verfahren. Hierzu wird durch die direkte oder indirekte Digitalisierung des zu restaurierenden Zahns bzw. der zu restaurierenden Zähne am Computer ein virtuelles Abbild der Zahnsituation erstellt, dann anhand dieses Abbilds am Computer ein Modell der Dentalrestauration oder Prothese konstruiert und dieses dann durch generative stereolithographische Fertigung hergestellt. Hierzu wird nach Erstellung des virtuellen Modells der herzustellenden Dentalrestauration oder Prothese die erfindungsgemäße Zusammensetzung durch selektive Lichteinstrahlung polymerisiert. Die Geometrie der Dentalrestauration oder Prothese kann schichtweise aufgebaut werden, indem nacheinander eine Vielzahl von dünnen Schichten mit gewünschtem Querschnitt polymerisiert wird. Nach dem schichtweisen Aufbau der Geometrie schließt sich üblicherweise eine Reinigung des Werkstücks durch Behandlung mit einem geeigneten Lösungsmittel, wie z.B. einem Alkohol, wie Ethanol oder Isopropanol, einem Keton oder einem Ester, und eine Nachvergütung durch Bestrahlung des Werkstücks mit einer geeigneten Wellenlänge, wie z.B. einer Bestrahlung mit einer Intensität von z.B. 25 $mW/cm^2$ bei 405 nm und gleichzeitig 130 mW bei 460 nm für 15 min, an, bei der das Werkstück mit Licht geeigneter Wellenlänge bestrahlt wird, gegebenenfalls unter gleichzeitiger Erwärmung oberhalb 50 °C, um den Restmonomergehalt weiter zu verringern und die mechanischen Eigenschaften zu verbessern.

Unter einer Nachvergütung von radikalischen Polymerisaten versteht man ein nachträgliche Wärmebehandlung und/oder zusätzliche Bestrahlung der Polymerisate zur Erhöhung des Doppelbindungs- bzw. Monomerumsatzes und damit zur Verbesserung der mechanischen und optischen Eigenschaften der Polymerisate.

Für eine Nachvergütung ist von Vorteil, zwei unterschiedliche Initiatoren einzusetzen, z.B. zwei Photoinitiatoren, die sich durch ihre Absorptionsbereiche unterscheiden, oder einen Photoinitiator und einen thermischen Initiator. Bevorzugt sind Mischungen von Photoinitiatoren für den UV-Bereich und den sichtbaren Bereich.

Die erfindungsgemäßen Zusammensetzungen enthalten mindestens einen Photoinitiator der Formel (I). Die Photoinitiatoren der Formel (I) gehören zu den UV-Photoinitiatoren, die aber bis in den sichtbaren Bereich hinein, d.h. bis etwa 450 nm, absorbieren. Bevorzugte UV-Photoinitiatoren zur Kombination mit den Photoinitiatoren der Formel (I) sind Acetophenone, z.B. 2,2-Diethoxy-1-phenylethanon, Benzoinether, wie z.B. Irgacure 651 (Dimethylbenzilketal), Hydroxyalkylphenylacetophenone, wie z.B. Irgacure 184 (1-Hydroxy-cyclohexyl-phenyl-keton), 2-Benzyl-2-(dimethylamino)-4'-morpholino-butyrophenon (Irgacure 369) und 1-Butanon-2-(dimethylamino)-2-(4-methylphenyl)-methyl-1-4-(4-morpholinyl)phenyl (Irgacure 379).

Die Photoinitiatoren der Formel (I) können auch mit Photoinitiatoren für den sichtbaren Bereich kombiniert werden. Geeignet sind α-Diketone und deren Derivate, wie 9,10-Phenanthrenchinon, 1-Phenyl-propan-1,2-dion, Diacetyl

oder 4,4'-Dichlorbenzil. Besonders bevorzugt werden Campherchinon (CQ) und 2,2-Dimethoxy-2-phenyl-acetophenon und ganz besonders bevorzugt α-Diketone in Kombination mit Aminen als Reduktionsmittel, wie z.B. 4-(Dimethylamino)-benzoesäureester (EDMAB), N,N-Dimethylaminoethylmethacrylat, N,N-Dimethylsym.-xylidin oder Triethanolamin, verwendet. Geeignete monomolekulare Photoinitiatoren für den sichtbaren Bereich sind auch Monoacyltrialkyl-, Diacyldialkyl- und Tetraacylgermanium sowie Tetraacylstannane, wie z.B. Benzoyltrimethylgermanium, Dibenzoyldiethylgermanium, Bis(4-methoxybenzoyl)diethylgermanium, Tetrakis(2-methylbenzoyl)germanium oder Tetrakis(mesitoyl)stannan. Dabei lassen sich auch Mischungen der verschiedenen Photoinitiatoren einsetzen, wie z.B. Bis(4-methoxybenzoyl)diethylgermanium in Kombination mit Campherchinon und 4-Dimethylaminobenzoesäureethylester. Erfindungsgemäß sind jedoch Zusammensetzungen bevorzugt, die neben den Photoinitiatoren der Formel (I) keine weiteren Photoinitiatoren enthalten, die im sichtbaren Wellenlängenbereich aktiv sind. Besonders bevorzugt sind Zusammensetzungen, die ausschließlich Photoinitiatoren der Formel (I) und keine weiteren Photoinitiatoren enthalten, weder für den sichtbaren noch für den UV-Bereich.

Als zusätzliche thermische Initiatoren sind Azoverbindungen, wie 2,2'-Azobis(isobuty-ronitril) (AIBN) oder Azobis-(4-cyanovaleriansäure), oder Peroxide, wie Dibenzoylperoxid, Dilauroylperoxid, tert-Butylperoctoat, tert-Butylperbenzoat oder Di-(tert-butyl)peroxid bevorzugt. Zur Beschleunigung der Initiierung mittels Peroxiden lassen sich auch Kombinationen mit aromatischen Aminen einsetzen. Bevorzugte Redoxsysteme sind: Kombinationen von Dibenzoylperoxid mit Aminen, wie N,N-Dimethyl-p-toluidin, N,N-Dihydroxyethyl-p-toluidin, p-Dimethylaminobenzoesäureethylester oder strukturverwandte Systeme.

Die Erfindung wird im Folgenden anhand von Figuren und Ausführungsbeispielen näher erläutert.

**Figur 1** zeigt die zeitliche Veränderung des b*-Werts gemäß CIE-Lab-Farbsystem von radikalisch polymerisierbaren Zusammensetzungen in Abhängigkeit von der Lagerungszeit in Wasser bei 50 °C. Die Zusammensetzungen enthalten 1,0 Gew.-% des Photoinitiators TPO und unterschiedliche Konzentrationen des Inhibitors MEHQ (Kurven von oben nach unten 100, 200, 300, 500 ppm).

**Figur 2** zeigt die zeitliche Veränderung des b*-Werts von Zusammensetzungen, die 1,0 Gew.-% des Photoinitiators TPO und unterschiedliche Konzentrationen des Inhibitors Pyrogallol enthalten (Kurven von oben nach unten 100, 200, 300, 500 ppm).

**Figur 3** veranschaulicht anhand der Zusammensetzung mit 300 ppm des Inhibitors MEHQ aus Figur 1 die Bestimmung des kinetischen Entfärbungsvektors. Gezeigt sind die Tangenten an die abfallende Flanke und an die Horizontale der Kurve. Die Gerade zwischen dem Koordinatenursprung und dem Schnittpunkt der Tangenten (Pfeil) entspricht dem kinetischen Entfärbungsvektor.

**Ausführungsbeispiele**

**Beispiel 1:**

*Synthese von (2,4-Dimethoxy-6-methylbenzoyl)diphenylphosphinoxid (K-274)*

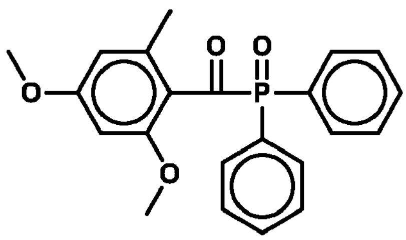

K-274

Triethylamin (0,79 g, 7.8 mmol) wurde langsam zu einer Mischung von 2,4-Dimethoxy-6-methylbenzaldehyd (1,40 g, 7,8 mmol) und Diphenylphosphinoxid (1,57 g, 7,8 mmol) in 50 mL THF zugetropft. Die Reaktionsmischung wurde dann für 72 h bei Raumtemperatur gerührt und das Lösungsmittel abgezogen. Das zurück gebliebene gelbliche Öl wurde dann in 50 Dichlormethan gelöst und mit aktivierten Mangan(IV)-dioxid (17,00 g, 0,20 mol) versetzt. Die gebildete Suspension wird dann für 48 h bei Raumtemperatur gerührt, über Celite abfiltriert und dann das Lösungsmittel abgezogen. Das so erhaltene Rohprodukt wurde säulenchromatographisch gereinigt (SiOz Säule, Elutionsmittel: Ethylacetat) und ergab 4,62 g (12,1 mmol; 44 % Ausbeute) als hochviskoses gelbliches Öl.

$^1$H-NMR ($CDCl_3$, 400 MHz): δ = 7.89 - 7.80 (m, 4H; Ar-H), 7.55 - 7.42 (m, 6H; Ar-H), 6.35 (br s, 1H; Ar-H), 6.30 (d, 1H; *J* = 2.1 Hz; Ar-H), 3.79 (s, 3H; $OCH_3$), 3.54 (s, 3H; $OCH_3$), 2.15 (s, 3H; $CH_3$).

$^{13}C$-NMR ($CDCl_3$, 100.6 MHz): δ = 211.1 (d, *J* = 84 Hz; C=O), 163.8 (Ar-C), 162.0 (d, *J* = 1 Hz; Ar-C), 140.4 (d, *J* = 4 Hz; Ar-C), 131.6 (d, *J* = 96 Hz; Ar-CH), 131.6 (d, *J* = 3 Hz; Ar-CH), 131.5 (d, *J* = 8 Hz; Ar-CH), 128.3 (d, *J* = 11 Hz; Ar-CH), 120.4 (d, *J* = 42 Hz; Ar-C), 109.1 (d, *J* = 2 Hz; Ar-CH), 96.0 (Ar-CH), 55.6 (O-$CH_3$), 55.4 (O-$CH_3$), 19.9 ($CH_3$).

$^{31}P$-NMR ($CDCl_3$, 162 MHz): δ = 17.48.

**Beispiel 2:**

*Synthese von (2,4,6-Trimethoxybenzovl)diphenylphosphinoxid (K-276)*

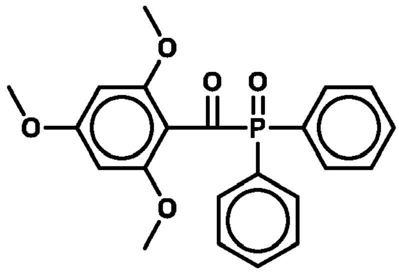

K-276

Analog zu Beispiel 1 wurden Triethylamin (5.06 g, 50.0 mmol), 2,4,6-Trimethoxybenzaldehyd (9.81 g, 50.0 mmol), Diphenylphosphinoxid (10.11 g, 50.0 mmol) in 150 mL THF gelöst und 6 h bei Raumtemperatur umgesetzt. Nach Entfernung des Lösungsmittels wurde der gelbliche Rückstand in 250 mL Dichloromethan aufgenommen, dazu wurde das aktivierte Mangan(IV)dioxid (86.94 g, 1.00 mol) zugegeben und die Suspension 24 h bei Raumtemperatur gerührt. Nach Aufarbeitung analog Beispiel 1 und Säulenchromatographie wurden 16.11 g (40.6 mmol; 81 % Ausbeute) des Produktes als gelblicher Feststoff erhalten.

$^{1}H$-NMR ($CDCl_3$, 400 MHz): δ = 7.88 - 7.79 (m, 4H; Ar-H), 7.53 - 7.38 (m, 6H; Ar-H), 6.07 (s, 2H; Ar-H), 3.78 (s, 3H; $OCH_3$), 3.60 (s, 6H; $OCH_3$).

$^{13}C$-NMR ($CDCl_3$, 100.6 MHz); δ = 207.3 (d, *J* = 88 Hz; C=O), 165.2 (Ar-C), 160.6 (Ar-C), 131.6 (d, *J* = 96 Hz; Ar-CH), 131.5 (d, *J* = 9 Hz; Ar-CH), 131.4 (d, *J* = 2 Hz; Ar-CH), 128.1 (d, *J* = 12 Hz; Ar-CH), 110.14 (d, *J* = 44 Hz; Ar-C), 90.9 (Ar-CH), 55.6 (O-CHs), 55.4 (O-CHs).

$^{31}P$-NMR ($CDCl_3$, 162 MHz): δ = 17.18.

**Beispiel 3:**

*Herstellung von polymerisierbaren Zusammensetzungen mit MEHQ*

Das Monomer UDMA (ca. 99 Gew.-%) wurde mit 1 Gew.-% jeweils eines der in Tabelle 1 aufgeführten Initiatoren und den in Tabelle 1 angegebenen Mengen des Inhibitors MEHQ homogen gemischt. Dazu wurden in einem Planetenmischer die Komponenten unter Rühren gelöst und bis zum Erreichen eines homogenen Gemisches weiter gerührt. Es ergaben sich stets vollkommen transparente Harze. Mit den Zusammensetzungen aus Tabelle 1 wurden in Metallformen Prüfkörper präpariert, die mit einer dentalen Lichtquelle (PrograPrint Cure, Fa. Ivoclar Vivadent AG, Schaan, Liechtenstein; Software: ProArt Print Splint, Jahr: 2020) beidseitig 2 x 1,5 Minuten bestrahlt und damit ausgehärtet. Es wurden vollkommen transparente Polymerisate erhalten.

Zur Bestimmung der Farbstabilität wurde der b*-Wert der Proben in Abhängigkeit von der Lagerzeit in Wasser bei 50 °C bestimmt. Exemplarisch sind die Ergebnisse für den Photoinitiator **TPO** und den Stabilisator **MEHQ** in Figur 1 dargestellt. Figur 1 zeig, dass die Monomerharze einen anfänglichen b*-Wert von ca. 6 aufweisen. Der b*-Wert steigt bei Beginn der Polymerisation sprunghaft an und fällt bei der anschließenden Lagerung wieder ab. Der Gelbwert der Harzmischung M-1, die 100 ppm des Inhibitors MEHQ enthält, steigt von 6 auf ca. 16 an. Erst nach Lagerung für ca. 450 Stunden (ca. 19 Tage) erreicht die Gelbfärbung wieder ihren Ausgangwert von ca. 6. Ab einem b*-Wert von ca. 4,5 ist die Gelbfärbung für das menschliche Auge nicht mehr wahrnehmbar. Diese Schwelle wird erst nach mehr als 600 Stunden (25 Tage) erreicht. Die Mischungen M-3 (300 ppm MEHQ) und M-4 (500 ppm MEHQ) zeigen demgegenüber nicht nur einen deutlich geringeren Anstieg, sondern auch einen sehr viel schnelleren Abfall des Gelbwerts. In beiden Fällen fällt der b*-Wert in weniger als 200 h (ca. 8 Tage) unter die Wahrnehmungsschwelle von 4,5.

Zur Bestimmung des Kinetischen Entfärbungsvektors der Harzmischungen wurde, wie in Figur 3 beispielhaft

gezeigt, jeweils eine Tangente an die abfallende Flanke und an die Horizontale der Kurve angelegt. Die Koordinaten des Schnittpunkts der Tangenten (Zeit und b*-Wert) sind in Tabelle 1 angegeben. Die Gerade zwischen dem Koordinatenursprung und dem Schnittpunkt der Tangenten entspricht dem Entfärbungsvektor (Pfeil).

**Tabelle: 1: Zusammensetzung der Werkstoffe mit dem Inhibitor MEHQ**

| Monomerharz | Monomer | Photoinitiator) | MeHQ[c] | Schnittpunkt der Tangenten | | | Länge Vektor[i] |
|---|---|---|---|---|---|---|---|
| | | | | b* | Zeit[h] [h] | Zeit[h] [Wochen] | |
| **M-1** | UDMA[a] | TPO[d] | 100 | 1,6 | 670 | 3,99 | 4,30 |
| **M-2** | UDMA | TPO | 200 | 1,6 | 460 | 2,74 | 3,17 |
| **M-3** | UDMA | TPO | 300 | 1,4 | 270 | 1,61 | 2,13 |
| **M-4** | UDMA | TPO | 500 | 1,1 | 205 | 1,22 | 1,64 |
| **M-5** | UDMA | TPO-L[e] | 100 | 1,9 | 665 | 3,96 | 4,39 |
| **M-6** | UDMA | TPO-L | 200 | 1,5 | 265 | 1,58 | 2,18 |
| **M-7** | UDMA | TPO-L | 300 | 1,7 | 150 | 0,89 | 1,92 |
| **M-8** | UDMA | TPO-L | 500 | 1,6 | 85 | 0,51 | 1,68 |
| **M-9** | UDMA | K-274[f] | 100 | 5,8 | 685 | 4,08 | 7,09 |
| **M-10** | UDMA | K-274 | 200 | 2,9 | 145 | 0,86 | 3,03 |
| **M-11** | UDMA | K-274 | 300 | 1,9 | 110 | 0,65 | 2,01 |
| **M-12** | UDMA | K-274 | 500 | 1,4 | 60 | 0,36 | 1,44 |
| **M-13** | UDMA | K-276[g] | 100 | 3,8 | 410 | 2,44 | 4,52 |
| **M-14** | UDMA | K-276 | 200 | 2,7 | 150 | 0,89 | 2,84 |
| **M-15** | UDMA | K-276 | 300 | 2,5 | 90 | 0,54 | 2,56 |
| **M-16** | UDMA | K-276 | 500 | 2,1 | 75 | 0,45 | 2,15 |

a) **UDMA:** ein Additionsprodukt aus 2-Hydroxyethylmethacrylat und 2,2,4-Trimethylhexamethylen-1,6-diisocyanat, Menge = Differenz zu 100 Gew.-%
b) Photoinitiatormenge: 1 Gew.-%
c) **MEHQ:** Hydrochinonmonomethylether, Mengenangabe in ppm
d) **TPO:** Diphenyl-(2,4,6-trimethylbenzoyl)phosphinoxid (Sigma-Aldrich)
e) **TPO-L:** 2,4,6-Trimethylbenzoylethoxylphenylphosphinoxid (IGM Resins)
f) **K-274:** (2,4-Dimethoxy-6-methylbenzoyl)diphenylphosphinoxid aus Bsp. 1
g) **K-276:** (2,4,6-Trimethoxybenzoyl)diphenylphosphinoxid aus Bsp. 2
h) Zeit: Lagerdauer der Probe bei 50°C
i) Betrag (=Länge) des kinetischen Entfärbungsvektors, berechnet als

$$\sqrt{(b^*)^2 + (Zeit\ [Wochen])^2}$$

**Beispiel 4:**

*Herstellung von polymerisierbaren Zusammensetzungen mit Pyrogallol*

Die in Tabelle 2 aufgeführten Komponenten wurden analog zu Beispiel 3 homogen miteinander gemischt und aus den Mischungen Prüfkörper hergestellt. Diese waren vollkommen transparent. Die Initiatorkonzentration betrug jeweils 1 Gew.-%. Der Inhibitor Pyrogallol wurde in den in Tabelle 2 genannten Mengen eingesetzt. Die Menge des Monomers UDMA betrug jeweils ca. 99 Gew.-%.

Zur Bestimmung der Farbstabilität wurde der b*-Wert der Proben in Abhängigkeit von der Lagerzeit in Wasser bei 50 °C bestimmt. Exemplarisch sind die Ergebnisse für den Photoinitiator **TPO** und den Stabilisator **Pyrogallol** in Figur 2 dargestellt. Die Mischung M-19 (300 ppm Pyrogallol) zeigt nur einen geringeren Anstieg des b*-Werts bei der Polymerisation, während der b*-Wert der Probe M-20 (500 ppm Pyrogallol) keinen Anstieg zeigt, sondern unmittelbar nach Polymerisationsbeginn abfällt. In beiden Fällen fällt der b*-Wert in weniger als 200 h (ca. 8 Tage) unter die Wahrnehmungsschwelle von 4,5. Die Probe M-20 erreicht diesen Wert schon nach ca. 25 h.

Die Bestimmung des Kinetischen Entfärbungsvektors erfolgte analog zu Beispiel 3. Die Ergebnisse werden in Tabelle 2 angegeben.

**Tabelle: 2: Zusammensetzung der Werkstoffe mit dem Inhibitor Pyrogallol**

| Monomerharz | Monomer | Photoinitiator[b)] | PGL[c)] | Schnittpunkt der Tangenten | | | Länge Vektor[i)] |
|---|---|---|---|---|---|---|---|
| | | | | b* | Zeit[h)] [h] | Zeit[h)] [Wochen] | |
| **M-17** | UDMA[a)] | TPO[d)] | 100 | 2,6 | 495 | 2,95 | 3,93 |
| **M-18** | UDMA | TPO | 200 | 2,3 | 385 | 2,29 | 3,25 |
| **M-19** | UDMA | TPO | 300 | 2,2 | 5 | 0,03 | 2,20 |
| **M-20** | UDMA | TPO | 500 | 2,3 | 5 | 0,03 | 2,30 |
| **M-21** | UDMA | TPO-L[e)] | 100 | 2,7 | 485 | 2,89 | 3,95 |
| **M-22** | UDMA | TPO-L | 200 | 2,6 | 462 | 2,75 | 3,78 |
| **M-23** | UDMA | TPO-L | 300 | 2,4 | 330 | 1,96 | 3,10 |
| **M-24** | UDMA | TPO-L | 500 | 2,1 | 32 | 0,19 | 2,11 |
| **M-25** | UDMA | K-276[g)] | 100 | 6,4 | 420 | 2,50 | 6,87 |
| **M-26** | UDMA | K-276 | 200 | 4,9 | 400 | 2,38 | 5,45 |
| **M-27** | UDMA | K-276 | 300 | 3,3 | 33 | 0,20 | 3,31 |
| **M-28** | UDMA | K-276 | 500 | 3,3 | 30 | 0,18 | 3,30 |

a) **UDMA:** ein Additionsprodukt aus 2-Hydroxyethylmethacrylat und 2,2,4-Trimethylhexamethylen-1,6-diisocyanat
b) Photoinitiatormenge: 1 Gew.-%
c) **PGL:** Pyrogallol, Mengenangabe in ppm
d) **TPO:** Diphenyl-(2,4,6-trimethylbenzoyl)phosphinoxid (Sigma-Aldrich)
e) **TPO-L:** 2,4,6-Trimethylbenzoylethoxylphenylphosphinoxid (IGM Resins)
f) **K-276:** (2,4,6-Trimethoxybenzoyl)diphenylphosphinoxid aus Bsp. 2
g) Zeit. Lagerdauer der Probe bei 50°C
h) Betrag (=Länge) des kinetischen Entfärbungsvektors, berechnet als

$$\sqrt{(b^*)^2 + (Zeit\ [Wochen])^2}$$

## Patentansprüche

1. Initiatorsystem für die radikalische Polymerisation, das mindestens ein Monoacylphosphinoxid der Formel (I) in der

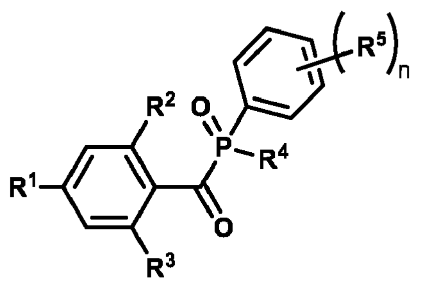

Formel (I)

$R^1$, $R^2$, $R^3$ unabhängig voneinander jeweils H, oder ein linearer oder verzweigter $C_1$-$C_{10}$-Alkylrest oder $OR^6$ sind,
$R^4$ ein linearer oder verzweigter $C_1$-$C_{10}$-Alkyl-, $C_1$-$C_{10}$-Alkoxy-, Cyclohexyl- oder ein Phenyl-, Methylphenyl- oder Mesityl-Rest ist,
$R^5$ ein linearer oder verzweigter $C_1$-$C_{10}$-Alkyl-Rest ist,
$R^6$ ein linearer oder verzweigter $C_1$-$C_6$-Alkyl-Rest ist, und
n 0, 1, 2 oder 3 ist,

und mindestens einen phenolischen Inhibitor enthält, **dadurch gekennzeichnet, dass** das Molverhältnis von Inhibitor zu Photoinitiator in einem Bereich von 0,002 bis 0,9 liegt.

2. Initiatorsystem nach Anspruch 1, wobei die Variablen die folgenden Bedeutungen haben:

   $R^1$, $R^2$, $R^3$ unabhängig voneinander jeweils ein linearer oder verzweigter $C_1$-$C_3$-Alkylrest oder $OR^6$, bevorzugt Methyl oder Methoxy,
   $R^4$ ein linearer oder verzweigter $C_1$-$C_5$-Alkyl-, $C_1$-$C_5$-Alkoxy-, Cyclohexyl- oder Phenyl-Rest, bevorzugt Ethoxy, Cyclohexyl oder Phenyl,
   $R^5$ ein linearer oder verzweigter $C_1$-$C_3$-Alkyl-Rest, bevorzugt Methyl,
   $R^6$ ein linearer oder verzweigter $C_1$-$C_3$-Alkyl-Rest, bevorzugt Ethyl, und
   n 0, 1 oder 3, bevorzugt 0,

3. Initiatorsystem nach Anspruch 1, wobei die Variablen die folgenden Bedeutungen haben:

   $R^1$, $R^2$, $R^3$ unabhängig voneinander jeweils H, ein linearer oder verzweigter $C_1$-$C_3$-Alkylrest oder $OR^6$, bevorzugt H, Methyl oder Methoxy, wobei besonders bevorzugt zwei der Reste $R^1$, $R^2$ und $R^3$ Methoxy sind und ein Rest H oder Methyl ist,
   $R^4$ ein linearer oder verzweigter $C_1$-$C_5$-Alkyl-, $C_1$-$C_5$-Alkoxy-, Cyclohexyl- oder Phenyl-Rest, bevorzugt tert,-Butyl, Ethoxy, Cyclohexyl oder Phenyl,
   $R^5$ ein linearer oder verzweigter $C_1$-$C_3$-Alkyl-Rest, bevorzugt Methyl,
   $R^6$ ein linearer oder verzweigter $C_1$-$C_3$-Alkyl-Rest, bevorzugt Methyl, und
   n 0, 1, 2 oder 3, ganz besonders bevorzugt 3,

   wobei dann, wenn $R^4$ kein Phenylrest ist, n vorzugsweise 0 ist, und wenn $R^4$ ein Phenylrest ist, n vorzugsweise 1, 2 oder 3 ist.

4. Initiatorsystem nach Anspruch 1, wobei die Variablen die folgenden Bedeutungen haben:

   $R^1$ H, ein linearer oder verzweigter $C_1$-$C_3$-Alkylrest oder $OR^6$, bevorzugt H, Methyl oder Methoxy,
   $R^2$, $R^3$ unabhängig voneinander jeweils H, ein linearer oder verzweigter $C_1$-$C_3$-Alkylrest oder $OR^6$, bevorzugt H, Methyl oder Methoxy, besonders bevorzugt Methyl oder Methoxy und am meisten bevorzugt Methoxy,
   $R^4$ ein linearer oder verzweigter $C_1$-$C_5$-Alkyl-, $C_1$-$C_5$-Alkoxy-, Cyclohexyl- oder Phenyl-Rest, bevorzugt Ethoxy, Cyclohexyl oder Phenyl,
   $R^5$ ein linearer oder verzweigter $C_1$-$C_3$-Alkyl-Rest, bevorzugt Methyl,
   $R^6$ ein linearer oder verzweigter $C_1$-$C_3$-Alkyl-Rest, bevorzugt Ethyl, und
   n 0, 1 oder 3, bevorzugt 0.

5. Initiatorsystem nach einem der Ansprüche 1 bis 4, wobei mindestens einer der Reste $R^1$, $R^2$ und $R^3$ $OR^6$ ist.

6. Initiatorsystem nach einem der Ansprüche 1 bis 4, bei der der phenolische Inhibitor (f) aus Hydrochinonmonomethylether, 2,6-Di-tert.-butyl-4-methyl-phenol, 2,6-Di-tert.-butylphenol, Pyrogallol, Hydrochinon, 2-tert.-Butylhydrochinon, 4-tert.-Butylbrenzcatechin, 6-tert.-Butyl-2,4-xylenol oder einer Mischung davon ausgewählt ist.

7. Radikalisch polymerisierbare Zusammensetzung, die ein Initiatorsystem gemäß einem der Ansprüche 1 bis 6 und mindestens ein Urethandi(meth)acrylat enthält.

8. Zusammensetzung nach Anspruch 7, die

   (a) 0,1 bis 8 Gew.-% mindestens eines Monoacylphosphinoxids der Formel (I),
   (b) 1 bis 99 Gew.-% mindestens eines urethangruppenhaltigen (Meth)acryl-atmonomers,
   (c) 0 bis 70 Gew.-% eines oder mehrerer aliphatischer, cycloaliphatischer, aromatischer, bicyclischer oder tricyclischer Mono(meth)acrylate,
   (d) 0 bis 50 Gew.-% eines oder mehrerer Di(meth)acrylats ohne Urethangruppen,
   (e) 0 bis 12 Gew.-% eines oder mehrerer Blockcopolymere und/oder Kern-Schale-Polymeren,
   (f) 200 bis 1200 ppm mindestens eines phenolischen Inhibitors,
   (g) 0 bis 50 Gew.-% eines oder mehrerer anorganischer und/oder organischer Füllstoffe,
   (h) 0 bis 30 Gew.-% an weiteren Additiven enthält,

jeweils bezogen auf die Gesamtmasse der Zusammensetzung.

**9.** Zusammensetzung nach Anspruch 8, die

(a) 0,30 bis 6,5 Gew.-% mindestens eines Monoacylphosphinoxids der Formel (I),
(b) 1 bis 80 Gew.-% mindestens eines urethangruppenhaltigen Monomers, vorzugsweise Urethandi(meth)acrylats,
(c) 0 bis 66 Gew.-%, vorzugsweise 5 bis 60 Gew.-%, eines oder mehrerer aliphatischer, cycloaliphatischer, aromatischer, bicyclischer oder tricyclischer Mono(meth)acrylate,
(d) 0 bis 40 Gew.-%, vorzugsweise 0 bis 25 Gew.-%, eines oder mehrerer Di(meth)acrylate ohne Urethangruppen,
(e) 0 bis 10 Gew.-%, vorzugsweise 0 bis 8 Gew.-%, eines oder mehrerer Blockcopolymere und/oder Kern-Schale-Polymeren,
(f) 300 bis 1000 ppm, vorzugsweise 300 bis 500 ppm, mindestens eines phenolischen Inhibitors,
(g) 0 bis 40 Gew.-%, vorzugsweise 0 bis 25 Gew.-% eines oder mehrerer anorganischer und/oder organischer Füllstoffe,
(h) 0 bis 25 Gew.-%, vorzugsweise 0,01 bis 10 Gew.-%, eines oder mehrerer weiterer Additive, die aus UV-Absorbern, optischen Aufhellern, Farbmitteln, Weichmachern und Thixotropieadditiven ausgewählt sind, enthält.

**10.** Zusammensetzung nach einem der Ansprüche 7 bis 9, wobei das urethangruppenhaltige (Meth)acrylatmonomer ein Urethandi(meth)acrylat ist, vorzugsweise ein Urethandi(meth)acrylat mit einem Urethangruppengehalt von 0,01 bis 5 mmol/g, besonders bevorzugt mit einem Urethangruppengehalt von 0,01 bis 5 mmol/g und einer Molmasse von kleiner 4000 g/mol, und ganz besonders bevorzugt ein Urethandi(meth)acrylat mit einem Urethangruppengehalt von 0,01 bis 5 mmol/g und einer Molmasse von kleiner 4000 g/mol, das 1 bis 8 Urethangruppen enthält.

**11.** Zusammensetzung nach einem der Ansprüche 7 bis 10, die einen kinetischen Entfärbungsvektor von kleiner 3,5, bevorzugt kleiner 3,0 und besonders bevorzugt kleiner 2,5 aufweist, wobei der Entfärbungsvektor auf die in der Beschreibung beschriebene Weise ermittelt wird.

**12.** Verwendung eines Initiatorsystems gemäß einem der Ansprüche 1 bis 6 zur Herstellung dentaler Formkörper mit einem b*-Wert unter 4,5 gemäß dem CIE-Lab-Farbsystem.

**13.** Verwendung einer Zusammensetzung gemäß einem der Ansprüche 7 bis 11 zur Herstellung dentaler Formkörper mit einem generativen Verfahren.

**14.** Verfahren zur Herstellung dentaler Formkörper, bei dem man

(i) durch die direkte oder indirekte Digitalisierung des zu restaurierenden Zahns bzw. der zu restaurierenden Zähne am Computer ein virtuelles Abbild der Zahnsituation erstellt,
(ii) man dann anhand dieses Abbilds am Computer ein Modell der Dentalrestauration oder Prothese konstruiert,
(iii) man durch schichtweise Polymerisation einer Zusammensetzung gemäß einem der Ansprüche 7 bis 11 mittels selektiver Lichteinstrahlung die Dentalrestauration oder Prothese schichtweise aufbaut.

**15.** Verwendung eines Inhibitors, der aus Hydrochinonmonomethylether, 2,6-Ditert.-butyl-4-methyl-phenol, 2,6-Ditert.-butylphenol, Pyrogallol, Hydrochinon, 2-tert.-Butylhydrochinon, 4-tert.-Butylbrenzcatechin, 6-tert.-Butyl-2,4-xylenol oder einer Mischung davon ausgewählt ist, zur Verhinderung von Verfärbungen radikalisch polymerisierbarer Zusammensetzungen, die durch Monoacylphosphinoxide der Formel (I) verursacht werden.

**Fig. 1**

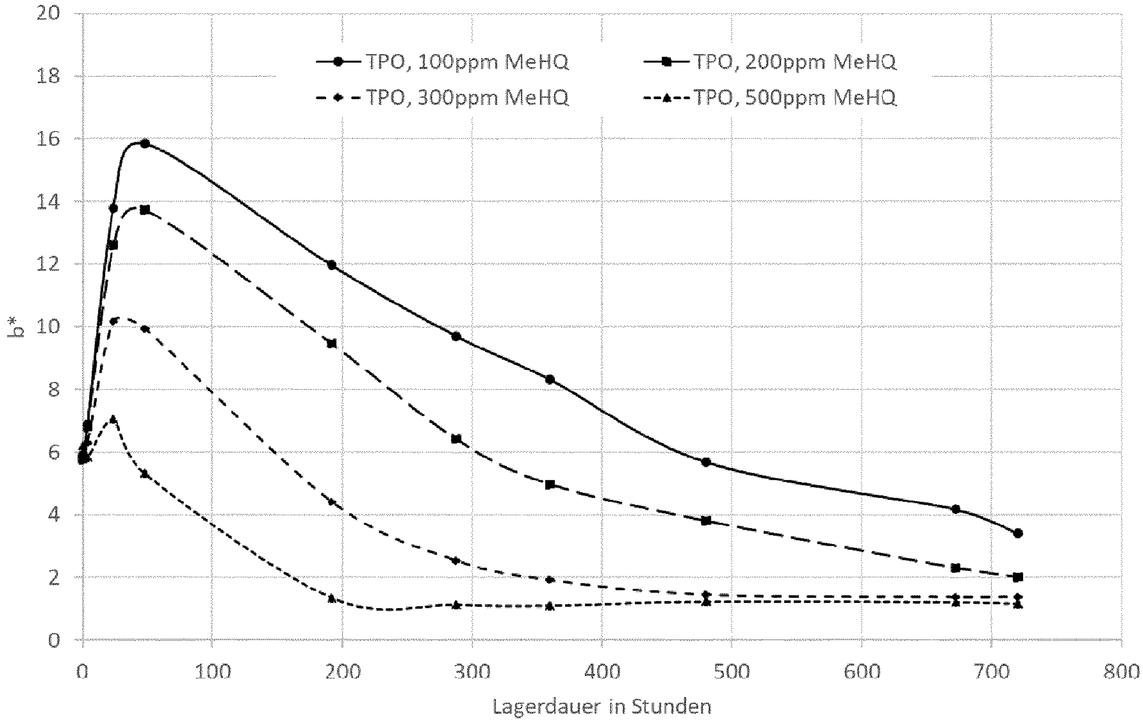
TPO, 100ppm MeHQ
TPO, 200ppm MeHQ
TPO, 300ppm MeHQ
TPO, 500ppm MeHQ
b*
Lagerdauer in Stunden
0
2
4
6
8
10
12
14
16
18
20
0
100
200
300
400
500
600
700
800

**Fig. 2**

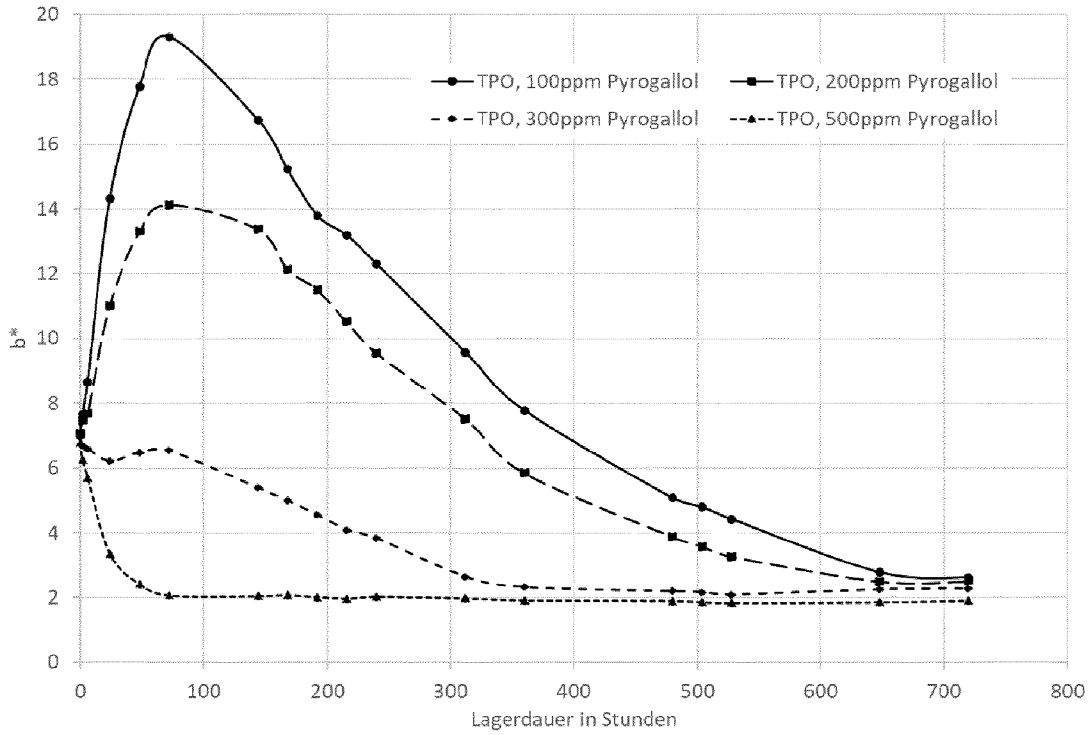
TPO, 100ppm Pyrogallol
TPO, 200ppm Pyrogallol
TPO, 300ppm Pyrogallol
TPO, 500ppm Pyrogallol
b*
Lagerdauer in Stunden
0
2
4
6
8
10
12
14
16
18
20
0
100
200
300
400
500
600
700
800

Fig. 3

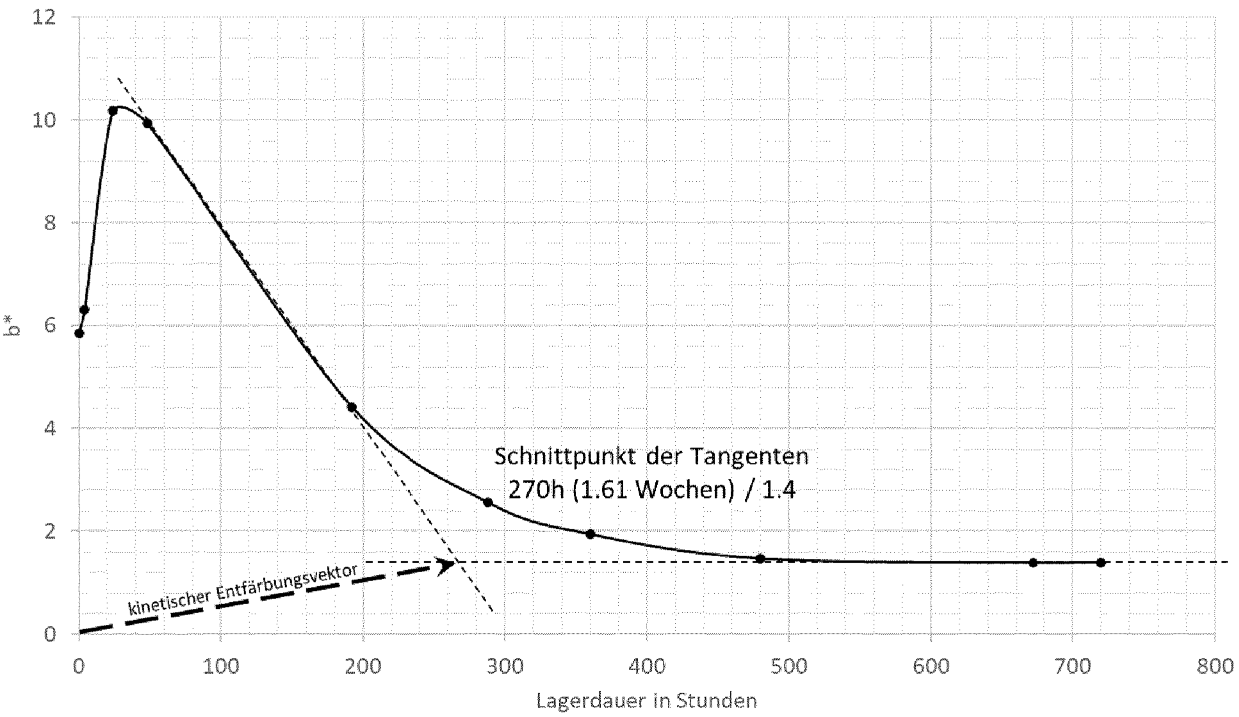
12
10
8
6
4
2
0
b*
Schnittpunkt der Tangenten
270h (1.61 Wochen) / 1.4
kinetischer Entfärbungsvektor
0
100
200
300
400
500
600
700
800
Lagerdauer in Stunden

Europäisches Patentamt
European Patent Office
Office européen des brevets

# EUROPÄISCHER RECHERCHENBERICHT

Nummer der Anmeldung
EP 23 18 2872

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X<br>A | WO 2020/177921 A1 (MUEHLBAUER TECH GMBH [DE]) 10. September 2020 (2020-09-10)<br>* TPO-enthaltendes Initiatorsystem für radikalische Polymerisation, enthaltend BHT (2,6-di-tert-butyl-4-methylphenol) as phenolischen Inhibitor und ein Urethandi(meth)acrylat, wobei das Verhältnis Inhibitor zur Monoacylphosphinoxid bei 0.07 liegt: Siehe Beispiel 1, Seite 28 *<br>* Verwendung des Initiatorsystems zur Herstellung dentaler Formkörper: siehe Ansprüche 6-10. *<br>* Verwendung für schichtweise Polymerisation durch 3D Druckverfahren im generativen Verfahren: Siehe Seite 28, letzter Abschnitt und Begin Seite 29 *<br>* Der phenolische Hilfsstoff wird als Stabilisator zugesetzt: Siehe Seite 13, Punkt d) *<br>-----<br>-/-- | 1-4,6-14<br>15 | INV.<br>C07F9/53<br>A61K6/62<br>A61K6/887<br>B33Y70/00<br>B33Y80/00<br>A61K6/16 |
| | | | RECHERCHIERTE SACHGEBIETE (IPC) |
| | | | C07F<br>A61K |

Der vorliegende Recherchenbericht wurde für alle Patentansprüche erstellt

2

| Recherchenort | Abschlußdatum der Recherche | Prüfer |
|---|---|---|
| Den Haag | 6. Dezember 2023 | Lange, Tim |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

Seite 1 von 4

Europäisches Patentamt
European Patent Office
Office européen des brevets

# EUROPÄISCHER RECHERCHENBERICHT

Nummer der Anmeldung
EP 23 18 2872

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X<br>A | DE 10 2021 124156 A1 (DENTONA AG [DE]) 30. Dezember 2021 (2021-12-30)<br>* Initiatorsystem für radikalische Polymerisation enthaltend Diphenyl(2,4,6-trimethylbenzoyl)phosphinoxid als Fotoinitiator und Urethandimethacrylat (siehe Beispiele 1 und 2 auf Seite 7), sowie 4-Methoxyphenol als Stabilisator (siehe Paragraph [36] und Anspruch 14), wobei die Menge an phenolischem Inhibitor (ganz bevorzugt 0.01-0.05 Gew.%) zu Fotoinitiator (1.16 - 1.11 in Beispielen 1-2) zwischen 0.009 und 0.04 beträgt *<br>* Verwendung des Initiatorsystems zur Herstellung dentaler Formkörper: siehe Anspruch 15 und Paragraph [16] *<br>* Verfahren zur Herstellung der medizinischen Formkörper durch schichtweise Polymerisation in additiven Verfahren: siehe Paragraph [39] *<br>* Der phenolische Hilfsstoff wird zur Stabilisation bzw. Inhibierung zugesetzt: Siehe Paragraph [36] *<br>-----<br>-/-- | 1-4,6-14<br>15 | |
| | | | RECHERCHIERTE SACHGEBIETE (IPC) |

Der vorliegende Recherchenbericht wurde für alle Patentansprüche erstellt

| Recherchenort | Abschlußdatum der Recherche | Prüfer |
|---|---|---|
| Den Haag | 6. Dezember 2023 | Lange, Tim |

2

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

Europäisches Patentamt
European Patent Office
Office européen des brevets

# EUROPÄISCHER RECHERCHENBERICHT

Nummer der Anmeldung
EP 23 18 2872

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 2011/139936 A2 (LEVIN LEANA [US]; LU HUI [US]) 10. November 2011 (2011-11-10)<br>* L-TPO-enthaltendes Initiatorsystem für radikalische Polymerisation, enthaltend BHT (2,6-di-tert-butyl-4-methylphenol) as phenolischen Inhibitor und ein Urethandi(meth)acrylat (UDMA), wobei das Verhältnis Inhibitor zur Monoacylphosphinoxid bei 0.18, 0.16, 0.25, 0.18 oder 0.19 liegt: Siehe Beispiele 1-5, Seiten 12-13 *<br>* Verwendung der Zusammensetzung zur Herstellung dentaler Formkörper: Siehe Ansprüche 1-9 *<br>----- | 1-4,6,7, 11-14 | |
| X<br>A | US 2018/000570 A1 (SUN BENJAMIN JIEMIN [US] ET AL) 4. Januar 2018 (2018-01-04)<br>* Initiatorsystem für radikalische Polymerisation enthaltend L-TPO(0.1g) and BHT (0.25 g x 1.3% = 0.0033 g), wobei das Verhältnis TPO zu BHT gleich 0.03 ist; Vewendung des Initiatorsystems zur Herstellung dentaler Formkörper (siehe Beispiel 4, Paragraph [54]) *<br>* Beispiele 6-15 *<br>* Verwendung der Zusammensetzungen zur Herstellung dentaler Formkörper, durch 3D Druckverfahren: siehe Beispiele 16-22, Ansprüche 1-20 *<br>----- | 1-4,6-13<br>15 | RECHERCHIERTE SACHGEBIETE (IPC) |
| X | US 2019/053883 A1 (SUN BENJAMIN J [US] ET AL) 21. Februar 2019 (2019-02-21)<br>* Initiatorsystem enthaltend 1.9 g L-TPO und 0.1 g BHT, wobei das Molverhältnis TPO zu BHT bei 0.07 liegt; Verwendung der Zusammensetzung für dentale Füllkörper: siehe Beispiel 5; Vewendung in 3D Druckverfahren: siehe Paragraph [113] *<br>-----<br>-/-- | 1-4,6,7, 11-14 | |

Der vorliegende Recherchenbericht wurde für alle Patentansprüche erstellt

2

| Recherchenort | Abschlußdatum der Recherche | Prüfer |
|---|---|---|
| Den Haag | 6. Dezember 2023 | Lange, Tim |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

Seite 3 von 4

Europäisches Patentamt
European Patent Office
Office européen des brevets

# EUROPÄISCHER RECHERCHENBERICHT

Nummer der Anmeldung
EP 23 18 2872

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 2022/168429 A1 (G C DENTAL IND CORP [JP]) 11. August 2022 (2022-08-11)<br>* Tabelle 1, Seite 16: Dentalkomposition enthaltend 2 Gew.% TPO and 1 Gew. % BHT *<br>* Alternativen zu TPO als Initiator der Photopolymerisation sind definiert in Paragraph [29] (der Uebersetzung), wie z. B. 2,4,6-Trimethoxybenzoyldiphenylphosphin oxid, Acyl phosphinoxide wie z. B. 6-Dimethylbenzoyldiphenylphosphin oxid oder 2,6-Dimethoxybenzoyldiphenylphosphin oxid *<br>----- | 1,5,8-10 | |

RECHERCHIERTE SACHGEBIETE (IPC)

Der vorliegende Recherchenbericht wurde für alle Patentansprüche erstellt

2

| Recherchenort | Abschlußdatum der Recherche | Prüfer |
|---|---|---|
| Den Haag | 6. Dezember 2023 | Lange, Tim |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.......................................................................
& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 23 18 2872

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

06-12-2023

| Im Recherchenbericht angeführtes Patentdokument | Datum der Veröffentlichung | Mitglied(er) der Patentfamilie | Datum der Veröffentlichung |
|---|---|---|---|
| WO 2020177921 A1 | 10-09-2020 | CA 3131556 A1 | 10-09-2020 |
| | | CN 113490478 A | 08-10-2021 |
| | | DE 102019105816 A1 | 10-09-2020 |
| | | EP 3934606 A1 | 12-01-2022 |
| | | JP 2022523572 A | 25-04-2022 |
| | | KR 20210137015 A | 17-11-2021 |
| | | US 2022151749 A1 | 19-05-2022 |
| | | WO 2020177921 A1 | 10-09-2020 |
| DE 102021124156 A1 | 30-12-2021 | KEINE | |
| WO 2011139936 A2 | 10-11-2011 | CA 2808332 A1 | 10-11-2011 |
| | | EP 2571479 A2 | 27-03-2013 |
| | | US 2011275035 A1 | 10-11-2011 |
| | | WO 2011139936 A2 | 10-11-2011 |
| US 2018000570 A1 | 04-01-2018 | CA 3029491 A1 | 04-01-2018 |
| | | EP 3478220 A1 | 08-05-2019 |
| | | ES 2834622 T3 | 18-06-2021 |
| | | JP 7010859 B2 | 26-01-2022 |
| | | JP 2019520912 A | 25-07-2019 |
| | | US 2018000570 A1 | 04-01-2018 |
| | | WO 2018005900 A1 | 04-01-2018 |
| US 2019053883 A1 | 21-02-2019 | CA 3026166 A1 | 28-12-2017 |
| | | EP 3472218 A1 | 24-04-2019 |
| | | JP 7036810 B2 | 15-03-2022 |
| | | JP 2019521188 A | 25-07-2019 |
| | | US 2017360534 A1 | 21-12-2017 |
| | | US 2019053883 A1 | 21-02-2019 |
| | | WO 2017223084 A1 | 28-12-2017 |
| WO 2022168429 A1 | 11-08-2022 | AU 2021425801 A1 | 24-08-2023 |
| | | CA 3210680 A1 | 11-08-2022 |
| | | CN 116829116 A | 29-09-2023 |
| | | EP 4289411 A1 | 13-12-2023 |
| | | JP 2022120710 A | 18-08-2022 |
| | | WO 2022168429 A1 | 11-08-2022 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 3020361 A1
- US 20180000570 A1
- US 10299896 B2
- US 20190053883 A1
- EP 3564282 A1
- EP 4085893 A1
- US 20080009557 A1


### In der Beschreibung aufgeführte Nicht-Patentliteratur


- **J. VIOHL** ; **K. DERMANN** ; **D. QUAST** ; **S. VENZ**. Die Chemie zahnärztlicher Füllungskunststoffe. Carl Hanser Verlag, 1986, 21-27
- **A. PEUTZFELDT**. Resin composites in dentistry: The monomer systems. *Eur. J. Oral. Sci.*, 1997, vol. 105, 97-116
- **N. MOSZNER** ; **T. HIRT**. New Polymer-Chemical Developments in Clinical Dental Polymer Materials: Enamel-Dentin Adhesives and Restorative Composites. *J. Polym. Sci. Part A: Polym. Chem.*, 2012, vol. 50, 4369-4402
- **A. GEBHARDT**. Generative Fertigungsverfahren. Carl Hanser Verlag, 2007, 77
- **I. GIBSON** ; **D.W. ROSEN** ; **B. STUCKER et al.** Additive Manufacturing Technologies. Springer Verlag, 2010, vol. 238
- **T. SUMIYOSHI** ; **W. SCHNABEL, W.** ; **HENNE, A.** Photolysis of acylphosphine oxides II: The influence of methyl substitution in benzoyldiphenylphosphine oxides. *J. Photochem.*, 1986, vol. 32, 119
- **C. DIETLIN** ; **T. T. TRINH** ; **S. SCHWEIZER** ; **B. GRAFF** ; **F. MORLET-SAVARY** ; **P.-A. NOIROT** ; **J. LALEVÉE**. Rational design of acyldiphenylphosphine oxides as photoinitiators of radical polymerization. *Macromolec*, 2019, vol. 52, 7886
- **H. DUAN** ; **K. LENG** ; **X. XU** ; **Q. LI** ; **D. LIU** ; **Y. HAN** ; **J. GAO** ; **Q. YU** ; **Z. WANG**. Monoacylphosphine oxides with substituents in the phosphonyl moiety as Norrish I photoinitiators: Synthesis, photoinitiation properties and mechanism. *J. Photochem. Photobiol. A: Chem*, 2021, vol. 421, 113517
- *CHEMICAL ABSTRACTS*, 150-76-5
- *CHEMICAL ABSTRACTS*, 128-37-0
- *CHEMICAL ABSTRACTS*, 128-39-2
- *CHEMICAL ABSTRACTS*, 87-66-1
- *CHEMICAL ABSTRACTS*, 123-31-9
- *CHEMICAL ABSTRACTS*, 1948-33-0
- *CHEMICAL ABSTRACTS*, 98-29-3
- *CHEMICAL ABSTRACTS*, 1879-09-0